(19) Europäisches Patentamt
European Patent Office
Office européen des brevets

(11) **EP 4 644 423 A1**

(12) **EUROPEAN PATENT APPLICATION**
published in accordance with Art. 153(4) EPC

(43) Date of publication:
**05.11.2025 Bulletin 2025/45**

(21) Application number: **23910908.5**

(22) Date of filing: **29.12.2023**

(51) International Patent Classification (IPC):
**C07K 16/28** (2006.01)   **C07K 16/46** (2006.01)
**A61K 39/00** (2006.01)

(52) Cooperative Patent Classification (CPC):
**A61K 39/00; C07K 16/28; C07K 16/46**

(86) International application number:
**PCT/CN2023/143105**

(87) International publication number:
**WO 2024/140994 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   **29.12.2022   CN 202211716944**

(71) Applicant: **Biyopharma Co., Ltd**
**Hangzhou, Zhejiang 310018 (CN)**

(72) Inventors:
• **HUANG, Mingyue**
  **Hangzhou, Zhejiang 310018 (CN)**
• **ZHAN, Xiaoxiao**
  **Hangzhou, Zhejiang 310018 (CN)**
• **LUO, Yi**
  **Hangzhou, Zhejiang 310018 (CN)**

(74) Representative: **Cooley (UK) LLP**
**22 Bishopsgate**
**London EC2N 4BQ (GB)**

(54) **FUSION PROTEIN**

(57)   Provided is a fusion protein. The fusion protein simultaneously blocks a calcitonin gene-related peptide (CGRP) pathway and a pituitary adenylate cyclase-activating polypeptide (PACAP) pathway, wherein the fusion protein comprises at least one single-domain antibody, at least one Fc fragment, and at least one extracellular domain fragment of pituitary adenylate cyclase 1 (PAC1 ECD). The fusion protein can simultaneously block the CGRP and PACAP pathways, thereby improving the response rate of a drug for treatment of migraine.

EP 4 644 423 A1

**Description**

**FIELD OF THE INVENTION**

The present invention relates to the field of biotechnology, and in particular to a fusion protein that simultaneously blocks a calcitonin gene-related peptide (CGRP) pathway and a pituitary adenylate cyclase-activating peptide (PACAP) pathway, and an application thereof.

**BACKGROUND OF THE INVENTION**

Migraine is a vascular neurological disease with 1.1 billion patients worldwide, and the number of patients is still increasing. The current global incidence rate is as high as 12-15%, and the incidence rate of severe chronic patients is 1.4-2.2%. It is one of the top 10 most disabling diseases in the world. The peak age for migraine onset is 37 years old, and it is three times more common in women than in men. An attack lasts 3 to 72 hours. In addition to unilateral headache, it is also accompanied by symptoms such as fear of light, fear of sound, nausea, and vomiting. Chronic patients often have cardiovascular diseases, mental disorders, depression, sleep disorders, and other problems. 90% of patients have their work, study, and social life affected; 30% of women have been affected by migraine; and 10% of children suffer from migraine. Currently, there are problems with both diagnosis and treatment of this disease, with only 5% of patients receiving correct diagnosis and treatment. Treatment methods include CGRP targeted drugs, 5-HTr agonists, calcium channel blockers, antiepileptic drugs, antidepressants, NSAIDs, nerve blockade/stimulation, and other nonspecific drugs.

The cause of migraine is unclear, so it is called primary headache. It is generally believed that some kind of stimulation of the central nervous system, such as CSD (Cortical Depression Spreading), causes the C-fiber terminals of the trigeminal nerve to release neurotransmitters such as calcitonin gene-related peptide (CGRP), pituitary adenylate cyclase-activating peptide (PACAP), and Substance C. These neurotransmitters bind to receptors on A$\delta$ fibers and trigger migraine directly or indirectly.

It is now known that there are at least two major signaling pathways involved in migraine: CGRP and PACAP. CGRP and PACAP are both neurotransmitters distributed in the nervous system and other tissues throughout the body. The receptors of both are class B GPCR neurotransmitters. They specifically bind to the receptors, resulting in an increase in the intracellular cAMP (cyclic adenosine monophosphate) concentration, and thereby achieving the purpose of signal transmission.

Many new drugs are being developed to block signaling in these two pathways. Antagonists of CGRP and its receptors have been successfully used in the treatment and prevention of migraine. Currently, there are four monoclonal antibodies on the European and American markets: Eptinezumab (Lunbeck), Erenumab (Amgen), Fremanezumab (Teva), and Galcanezumab (Eli Lilly). There are also small molecules against CGRP receptors, including Atogepant (AbbVie), Ubrogepent (AbbVie), and Rimegepent (Pfizer), which are available on the European and American markets. In contrast, antagonists of PACAP and PAC1 are still under development. Clinical studies have shown that administering PACAP to healthy people can induce migraine-like headache attacks. PACAP has also been found to be higher in the blood of chronic migraine patients than in healthy people. These studies have all demonstrated that PACAP is closely related to migraine.

According to the latest statistics published in the New England Journal of Medicine, the response rates of the main drugs used to treat migraine are very low. The response rate of preventive CGRP monoclonal antibodies is only 20-24%, while that of small molecule CGRP receptor antagonists is only about 10-14%. One of the reasons for the low response rate of patients to drugs is that the onset of migraine involves multiple signaling pathways. Therefore, it is necessary to develop dual-function or multifunctional drugs to achieve the maximum therapeutic effect and provide effective treatment for the majority of migraine patients, especially those with refractory migraine.

**SUMMARY OF THE INVENTION**

The present invention provides a fusion protein that simultaneously blocks CGRP and PACAP pathways, and the fusion protein can improve the response rate of drugs for treating migraine.

In one aspect, the present invention provides a single-domain antibody for blocking the calcitonin gene-related peptide (CGRP) pathway, wherein the single-domain antibody comprises a CDR1 as shown in the amino acid sequence of SEQ ID NO: 2, 7, 12, 17, 22, 27, 32, 37, 42, 47, 52, or 57 or any variant thereof, a CDR2 as shown in the amino acid sequence of SEQ ID NO: 3, 8, 13, 18, 23, 28, 33, 38, 43, 48, 53, or 58 or any variant thereof, and a CDR3 as shown in the amino acid sequence of SEQ ID NO: 4, 9, 14, 19, 24, 29, 34, 39, 44, 49, 54, or 59 or any variant thereof.

In another aspect, the present invention provides a fusion protein, which simultaneously blocks the calcitonin gene-related peptide (CGRP) pathway and the pituitary adenylate cyclase-activating peptide (PACAP) pathway, wherein the fusion protein comprises at least one single-domain antibody, at least one Fc fragment, and at least one extracellular domain fragment of pituitary adenylate cyclase 1 (PAC1 ECD).

The fusion protein comprises one peptide chain, the structure of which is that one PAC1 ECD is connected to the C-terminus of an Fc fragment via a linker, and the N-terminus of the Fc fragment is fused to the C-terminus of a single-domain antibody;

optionally, the fusion protein comprises two peptide chains, the structure of which is that one PAC1 ECD is connected to the C-terminus of an Fc fragment via a linker, and the N-terminus of the Fc fragment is fused to the C-terminus of a single-domain antibody, and two PAC1 ECDs of the two peptide chains are connected via a linker;

and optionally, the fusion protein comprises three peptide chains, the structure of which is that one PAC 1 ECD is connected to the C-terminus of an Fc fragment via a linker, and the N-terminus of the Fc fragment is fused to the C-terminus of a single-domain antibody, and three PAC1 ECDs of the three peptide chains are connected to each other via a linker.

In another aspect, the present invention provides nucleic acids encoding the above-mentioned single-domain antibodies and fusion proteins.

In another aspect, the present invention provides a vector comprising the above nucleic acids.

In another aspect, the present invention provides cells comprising the above nucleic acids or vector.

In another aspect, the present invention provides a pharmaceutical composition or kit, which comprises the above-mentioned single-domain antibody, fusion protein, and a pharmaceutically acceptable vector, diluent, or excipient.

In another aspect, the present invention provides a use of the above-mentioned single-domain antibody, fusion protein, nucleic acids, vector, and/or cells and/or pharmaceutical composition or kit in the preparation of a drug for treating diseases related to CGRP and PACAP pathway activation.

The fusion protein provided by the present invention can block two signal pathways at the same time, improve the response rate of migraine patients to drugs, and provide effective treatment methods for a large number of migraine patients, especially those with refractory migraine.

## BRIEF DESCRIPTION OF THE DRAWINGS

Other features, objectives, and advantages of the present invention will become more apparent from the following detailed description of non-limiting embodiments with reference to the accompanying drawings. The accompanying drawings are only for the purpose of illustrating specific embodiments and are not to be considered limitations to the present invention. In the accompanying drawings:

FIG. 1 shows an electrophoresis result of RC-Fc.

FIG. 2 shows that the reconstructed CGRP receptor extracellular region of the present invention retains the ability to bind to its ligand CGRP, but does not bind to the irrelevant polypeptide PACAP.

FIG. 3 shows CGRP-induced cAMP production under different SK-N-MC cell numbers.

FIG. 4 shows electrophoresis results of 42wtFc, 42hv10Fc, 42hv11Fe, and 42hv12Fc.

FIG. 5 shows that VHH No. 42 after humanization and Fc fusion retains the ability to inhibit the cellular cAMP production.

FIG. 6 shows the gene structure of human PAC1. Among them, the vertical bars are exons.

FIG. 7 shows three ligands of PAC1, including PACAP (PACAP38), PACAP27, and VIP.

FIG. 8 shows electrophoresis results of PAC1 ECD-Fc and PACs1 ECD-Fc.

FIG. 9 shows a comparison of the abilities of PAC1 ECD-Fc and PAC1s ECD-Fc to bind to PACAP in vitro.

FIG. 10 shows the structure of BY003.

FIG. 11 shows the working principle of BY003.

FIG. 12 shows an electrophoresis result of BY003. Among them, from left to right are the electrophoresis result diagrams of 42hv10FeNA-1×PAC1s ECD, 42hv10FcNA-2×PAC1s ECD, and 42hv10FcNA-3×PAC1s ECD, which correspond to the cases of one, two, or three PAC1s ECDs, respectively.

FIG. 13 shows the CGRP standard curves of three CGRPr stable cell lines.

FIG. 14 shows that BY003 inhibits CGRP-induced cAMP production in CHO-hCGRPrC3 cells.

FIG. 15 shows the PACAP standard curves of six PAC1 stable cell lines.

FIG. 16 shows that BY003 blocks PACAP signaling at the cellular level.

**DETAILED DESCRIPTION OF THE INVENTION**

I. Definitions

In the present invention, unless otherwise specified, the scientific and technical terms used herein have the meanings commonly understood by those skilled in the art. Furthermore, the terms and laboratory procedures related to protein and nucleic acid chemistry, molecular biology, cell and tissue culture, microbiology, and immunology used herein are terms and common procedures widely used in corresponding fields. Meanwhile, in order to better understand the present invention, definitions and explanations of relevant terms are provided below.

The term "fusion" or "fused" when applied to amino acid sequences (e.g., peptides, polypeptides, or proteins) refers to the combination of two or more amino acid sequences into a non-naturally occurring single amino acid sequence, for example, by means of chemical bonding or recombination. The fused amino acid sequence can be produced by the recombination of two genes encoding polynucleotide sequences, and can be expressed by introducing a construct containing the recombinant polynucleotide into a host cell.

The terms "polypeptide," "peptide," and "protein" are used interchangeably in the present disclosure to refer to a polymer of amino acid residues, or an aggregate of polymers of multiple amino acid residues. It covers natural or artificial proteins, protein fragments, and polypeptide analogs having protein sequences. These terms apply to amino acid polymers in which one or more amino acid residues are artificial chemical simulants of corresponding naturally occurring amino acids, as well as to naturally occurring amino acid polymers and non-naturally occurring amino acid polymers. Polypeptides may be monomeric or polymeric.

With respect to antibody chain polypeptide sequences, the phrase "substantially identical" may be understood as referring to antibody chains that exhibit at least 60%, 65%, 70%, 75%, 80%, 85%, 90%, 95%, 96%, 97%, 98%, 99%, or higher sequence identity to a reference polypeptide sequence. With respect to nucleic acid sequences, this term may be understood to mean nucleotide sequences that exhibit a sequence identity at least greater than 60%, 65%, 70%, 75%, 80%, 85%, 90%, 95%, 96%, 97%, 98%, 99%, or higher to a reference nucleic acid sequence.

Sequence "identity" or "identity" has a meaning that is commonly recognized in the art, and the percentage of sequence identity between two nucleic acid or polypeptide molecules or regions can be calculated using a published techniques. Sequence identity can be measured along the entire length of a polynucleotide or polypeptide, or along a region of the molecule (see, for example, Computational Molecular Biology, Lesk, A.M., ed., Oxford University Pres, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods to measure the identity between two polynucleotides or polypeptides, the term "identity" is well known to those of skill (Carrillo, H. & Lipman, D., SIAM J Applied Math 48: 1073 (1988)).

The term "antibody," also referred to as "immunoglobulin" (Ig) in the art, refers to proteins built from paired heavy and light polypeptide chains; there are various Ig isotypes, including IgA, IgD, IgE, IgG, and IgM. When antibodies are correctly folded, each chain is folded into many distinct globular domains connected by more linear peptide sequences. For example, immunoglobulin light chains are folded into variable (VL) and constant (CL) domains, while heavy chains are folded into variable (VH) and three constant (CH1, CH2, CH3) domains. The interaction between the heavy and light chain variable domains (VH and VL) results in the formation of the antigen binding region (Fv).

The term "single-domain antibody" (sdAb) refers to an antibody that contains only a single antibody variable region (VH/VL). Like conventional antibodies, single-domain antibodies can selectively bind to specific antigens. The molecular weight of a single-domain antibody is only 12-15 kDa, which is much smaller than a common antibody (150-160 kDa) consisting of two heavy chains and two light chains. The term "heavy-chain antibody," "heavy-chain single-domain antibody," or "nanosingle chain antibody" comprises a single variable domain VHH (variable domain of heavy chain of heavy-chain antibody) and two constant domains (CH2 and CH3). Importantly, the cloned and isolated VHH domains are fully stable polypeptides that possess the full antigen binding capacity of the original heavy-chain antibody. This type of antibodies naturally lacks light chains compared with other antibodies. This type of single-domain antibodies is discovered in camelid animals. Single-domain antibodies have the advantages of stable structure, small molecule, good solubility, tolerance to various adverse environments, and easy humanization. They have been widely used in miniaturized genetic engineering antibody research, new drug development, and disease diagnosis and treatment. Examples of single-domain antibodies include those derived from camelid animals (alpacas and camels) and cartilaginous fish (e.g., nurse sharks) and those derived from human and mouse antibodies by recombinant methods. As used herein, the term "single-domain antibody" includes those sdAbs isolated directly from VH, VHH, VL, or VNAR libraries of any source by phage display or other techniques, sdAbs derived from the aforementioned sdAbs, recombinantly produced sdAbs, and those sdAbs produced by further modification of such sdAbs (by humanization, affinity maturation, stabilization, solubilization, camelization, or other methods of antibody engineering). The present disclosure further relates to homologues, derivatives, or fragments that retain the antigen binding function and specificity of sdAbs.

The term "linker" refers to a peptide comprising one or more amino acids, typically about 2-20 amino acids. Linkers are known in the art or described herein. In some embodiments, the linker is a flexible polypeptide. The flexible polypeptide is composed of flexible amino acids, and the flexible amino acids are selected from at least one of Gly, Ser, Ala, and Thr. Suitable non-immunogenic linker peptides are, for example, (G4S)n, (SG4)n, or G4(SG4)n peptide linkers, i.e., the peptide is selected from the group consisting of GGGGS, GGGGSGGGGS, SGGGGSGGGG, GGGGGSGGGGSSGGGGS, GGGGSGGGGSGGGGS GGGGSGGGGSGGGG, GGGGSGGGGSGGGGSGGGGS, GGGGSGGGGSGGGSGGGGS, GSGSGSGS, GGSGSGSG, GGSGSG, GGSG.

The term "CDR" refers to a complementarity-determining region, and each heavy chain and light chain of a known antibody molecule has three CDRs. CDR is also referred to as a hypervariable region and is present in the variable region of each heavy chain and light chain of an antibody, with sites of very high variability in the primary structure of the CDR. In the present specification, a heavy-chain CDR is represented by CDR1, CDR2, and CDR3 from an amino terminal of an amino terminal sequence of the heavy chain, and a light chain CDR is represented by CDR1, CDR2, and CDR3 from an amino terminal of an amino terminal sequence of the light chain. These sites are located adjacent to one another in the tertiary structure and determine the specificity of an antigen to which the antibody binds.

"Specific binding" or "immunospecifically bind" with respect to an antibody or antigen-binding fragment thereof is used interchangeably herein and refers to the ability of an antibody or antigen-binding fragment to form one or more non-covalent bonds with an alloantigen through non-covalent interactions between the antibody combining sites of the antibody and the antigen. The antigen may be an isolated antigen or present in a tumor cell. Typically, an antibody that immunospecifically binds (or specifically binds) an antigen binds the antigen with an affinity constant Ka of about or $1 \times 10^7$ M-1 or $1 \times 10^8$ M-1 or greater (or a dissociation constant (Kd) of $1 \times 10$-7 M or $1 \times 10$-8 M or less). Affinity constants can be determined by standard kinetic methods of antibody reactions, e.g., immunoassays, surface plasmon resonance (SPR) (Rich and Myszka (2000) Curr. Opin. Biotechnol 11:54; Englebienne (1998) Analyst. 123:1599), isothermal titration calorimetry (ITC), or other kinetic interaction assays known in the art; see also U.S. Pat. No. 7,229,619 for a description of exemplary SPR and ITC methods for calculating the binding affinity of an antibody). Instruments and methods for real-time detection and monitoring of binding rates are known and commercially available (see, BiaCore 2000, Biacore AB, Upsala, Sweden and GE Healthcare Life Sciences; Malmqvist (2000) Biochem. Soc. Trans. 27:335).

The term "binding affinity" or "affinity" refers to the strength of the sum total of non-covalent interactions between a single binding site of a molecule (e.g., an antibody) and its binding partner (e.g., an antigen). Unless otherwise indicated, as used herein, the "binding affinity" refers to the intrinsic binding affinity that reflects a 1:1 interaction between members of a binding pair (e.g., an antibody and an antigen). The affinity of a molecule X for its partner Y can generally be represented by a dissociation constant (Kd). Affinity can be measured by common methods known in the art, including those described herein. A low-affinity antibody generally binds an antigen slowly and tends to dissociate readily, whereas a high-affinity antibody generally binds an antigen quickly and tends to remain bound longer. Various methods of measuring binding affinity are known in the art.

The term "inhibit" or "neutralize" as used with respect to the biological activity of a fusion protein of the present disclosure refers to the ability of a polypeptide to substantially antagonize, inhibit, prevent, limit, slow, destroy, eliminate, stop, reduce, or reverse, for example, the progression or severity of that which is inhibited (including but not limited to a biological activity).

The terms "nucleic acid" and "nucleic acid molecule" refer to an oligomer or polymer comprising at least two linked nucleic acids or nucleic acid derivatives, including deoxyribonucleic acid (DNA) and ribonucleic acid (RNA) typically linked together by phosphodiester bonds. As used herein, the term "nucleic acid molecule" is intended to include DNA molecules and RNA molecules. A nucleic acid molecule may be single-stranded or double-stranded, and may be cDNA.

The term "vector" includes vectors capable of expressing DNA that is operably linked to a regulatory sequence such as promoter regions capable of affecting the expression of such DNA fragments. Such additional segments may include promoter and terminator sequences, and optionally may include one or more origins of replication, one or more selectable markers, an enhancer, a polyadenylation signal, and the like. Expression vectors are generally derived from plasmids or viral DNAs, or elements that may contain both. Thus, an expression vector refers to a recombinant DNA or RNA construct, such as a plasmid, phage, recombinant virus, or other vectors, which, when introduced into an appropriate host cell, results in the expression of the cloned DNA. Appropriate expression vectors are well known to those skilled in the art and include expression vectors replicable in eukaryotic cells and/or prokaryotic cells as well as expression vectors that remain episomal or are integrated into the host cell genome.

The term "pharmaceutical composition" refers to such preparation that is present in a form permitting the biological activity of the active ingredient contained therein to be effective, and contains no additional ingredients that are unacceptably toxic to a subject to which the preparation would be administered.

The term "pharmaceutically acceptable vector" refers to one or more nontoxic materials that are administered with a therapeutic agent and do not interfere with the biological activity of the active ingredient, including but not limited to buffers, preservatives, compatible vectors, diluents, adjuvants (e.g., Freund's adjuvant (complete and incomplete)), excipients, vehicles, and optionally other additives or encapsulating substances. The pharmaceutical vectors suitable for the present disclosure may be conventional pharmaceutical preparation excipients, as well as compositions and preparations suitable for delivering the disclosed neutralizing antibodies. In general, the nature of a vector depends on the particular mode of administration being employed. For example, parenteral preparations usually include injectable liquids that include pharmaceutically and physiologically acceptable liquids such as water, physiological saline, balanced salt solutions, glucose solutions, glycerol, etc. as a vehicle. For solid compositions (e.g., powder, pill, tablet, or capsule form), conventional non-toxic solid vehicles may include, for example, pharmaceutical-grade mannitol, lactose, starch, or magnesium stearate. In addition to biologically neutral vehicles, an administered pharmaceutical composition may also contain a small amount of non-toxic auxiliary substances, such as wetting or emulsifying agents, preservatives, and pH buffers, such as sodium acetate or sorbitan monolaurate.

The term "treating" refers to a subject suffering from a disease or condition indicating that the subject's symptoms are partially or completely alleviated, or remain unchanged after treatment. Thus, the treatment includes prevention, therapy, and/or cure. Prevention refers to keeping an underlying disease at bay and/or preventing symptoms from getting worse or a disease from developing. Treatment further comprises any pharmaceutical use of any of the provided antibodies or antigen-binding fragments thereof as well as compositions provided herein.

Also provided are "conservative sequence modifications" of the sequences described in the sequence listing described herein, i.e., nucleotide and amino acid sequence modifications that do not eliminate the binding of the antibody encoded by the nucleotide sequence or containing the amino acid sequence to the antigen. These conservative sequence modifications include conservative nucleotides and amino acid substitutions as well as nucleotide and amino acid additions and deletions. For example, modifications can be introduced into the sequence listing described herein by standard techniques known in the art (such as site-directed mutagenesis and PCR-mediated mutagenesis). The conservative sequence modifications include conservative amino acid substitutions, in which an amino acid residue is replaced with an amino acid residue having a similar side chain. Families of amino acid residues having similar side chains have been defined in the art. These families include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine, tryptophan), amino acids with nonpolar side chains (e.g., alanine, valine, leucine, isoleucine, proline, phenylalanine, methionine), amino acids with β-branched side chains (e.g., threonine, valine, isoleucine), and amino acids with aromatic side chains (e.g., tyrosine, phenylalanine, tryptophan, histidine). Therefore, a predicted nonessential amino acid residue in an anti-BCMA antibody is preferably replaced with another amino acid residue from the same side chain family. Methods for identifying conservative substitutions of nucleotides and amino acids that do not eliminate antigen binding are well known in the art (e.g., see Brummell et al., Biochem. 32: 1180-1187 (1993): Kobayashi et al., Protein Eng. 12(10): 879-884 (1999); Burks et al., Proc.Natl.Acad.Sci.USA 94: 412-417 (1997)). In some embodiments, no more than 1, 2, 3, 4, 5, 6, 7, 8, 9, or 10 amino acid changes are present in one or more or all three heavy-chain CDRs. Preferably, the above-mentioned amino acid changes are amino acid substitutions, preferably conservative substitutions. In some embodiments, an antibody variant has at least greater than 60%, 65%, 70%, 75%, 80%, 85%, 90%, 95%, 96%, 97%, 98%, 99%, or higher sequence identity with the parent antibody over the target antibody sequence region. Exemplary substitutions are shown in Table 1 below:

Table 1 Conservative amino acid substitutions

| Original residue | Exemplary substitutions | Preferred conservative substitutions |
|---|---|---|
| Ala (A) | Val (V): Leu (L): Ile (I) | Val (V) |
| Arg (R) | Lys (K); Gln (Q); Asn (N); His (H) | Lys (K) |
| Asn (N) | Gln (Q); His (H); Asp (D); Lys (K); Arg (R) | Gln (Q) |
| Asp (D) | Glu (E);Asn (N) | Glu (E) |

(continued)

| Original residue | Exemplary substitutions | Preferred conservative substitutions |
|---|---|---|
| Cys (C) | Ser (S); Ala (A) | Ser (S) |
| Gln (Q) | Asn (N); Glu (E) | Asn (N) |
| Glu (E) | Asp (D): Gln (Q) | Asp (D) |
| Gly (G) | Ala (A) | Ala (A) |
| His (H) | Asn (N); Gln (Q); Lys (K); Arg (R) | Arg (R) |
| Ile (I) | Leu (L); Val (V); Met (M); Ala (A); Phe (F); Norleucine Nle | Leu (L) |
| Leu (L) | Norleucine Nle: Ile (I): Val (V): Met (M); Ala (A); Phe (F) | Ile (I) |
| Lys (K) | Arg (R): Gln (Q); Asn (N); His (H) | Arg (R) |
| Met (M) | Leu (L); Phe (F): Ile (I) | Leu (L) |
| Phe (F) | Trp (W); Leu (L); Val (V); Ile (I); Ala (A); Tyr (Y) | Tyr (Y) |
| Pro (P) | Ala (A) | Ala (A) |
| Ser(S) | Thr (T) | Thr (T) |
| Thr (T) | Val (V): Ser (S) | Ser (S) |
| Trp (W) | Tyr (Y); Phe (F) | Tyr (Y) |
| Tyr (Y) | Trp (W); Phe (F); Thr (T); Ser (S) | Phe (F) |
| Val (V) | Ile (I): Leu (L); Met (M); Phe (F); Ala (A); Norleucine Nle | Leu (L) |

## II. DETAILED DESCRIPTION OF THE INVENTION

In one aspect, the present invention provides a single-domain antibody for blocking the calcitonin gene-related peptide (CGRP) pathway, wherein the single-domain antibody comprises a CDR1 as shown in the amino acid sequence of SEQ ID NO: 2, 7, 12, 17, 22, 27, 32, 37, 42, 47, 52, or 57 or any variant thereof, a CDR2 as shown in the amino acid sequence of SEQ ID NO: 3, 8, 13, 18, 23, 28, 33, 38, 43, 48, 53, or 58 or any variant thereof, and a CDR3 as shown in the amino acid sequence of SEQ ID NO: 4, 9, 14, 19, 24, 29, 34, 39, 44, 49, 54, or 59 or any variant thereof.

In some preferred embodiments, the above-mentioned single-domain antibody comprises CDR1 as shown in the amino acid sequence of SEQ ID NO: 12 or any variant thereof, CDR2 as shown in the amino acid sequence of SEQ ID NO: 13 or any variant thereof, and CDR3 as shown in the amino acid sequence of SEQ ID NO: 14 or any variant thereof.

In some embodiments, the single-domain antibody comprises a variable region as shown in the amino acid sequence of SEQ ID NO: 1, 6, 11, 16, 21, 26, 31, 36, 41, 46, 51, 56, 61, 62, or 63 or any variant thereof.

In some preferred embodiments, the single-domain antibody comprises a variable region as shown in the amino acid sequence of SEQ ID NO: 61, 62, or 63 or any variant thereof.

In some preferred embodiments, the single-domain antibody comprises a variable region as shown in the amino acid sequence of SEQ ID NO: 61 or any variant thereof.

In some preferred embodiments, the amino acids in the above-mentioned single-domain antibodies have conservative substitutions, and the number of conservatively substituted amino acids does not exceed 1, 2, 3, 4, 5, 6, 7, 8, 9, or 10.

In one aspect, the present invention provides a fusion protein, which simultaneously blocks the calcitonin gene-related peptide (CGRP) pathway and the pituitary adenylate cyclase-activating peptide (PACAP) pathway, wherein the fusion protein comprises at least one single-domain antibody, at least one Fc fragment, and at least one extracellular domain fragment of pituitary adenylate cyclase 1 (PAC1ECD).

In some embodiments, the fusion protein comprises one peptide chain, the structure of which is that one PAC1 ECD is connected to the C-terminus of an Fc fragment via a linker, and the N-terminus of the Fc fragment is fused to the C-terminus of a single-domain antibody.

In some embodiments, the fusion protein comprises two peptide chains, the structure of which is that one PAC1 ECD is connected to the C-terminus of an Fc fragment via a linker, and the N-terminus of the Fc fragment is fused to the C-terminus of a single-domain antibody, and two PAC1 ECDs of the two peptide chains are connected via a linker.

In some embodiments, the fusion protein comprises three peptide chains, the structure of which is that one PAC1 ECD is connected to the C-terminus of an Fc fragment via a linker, and the N-terminus of the Fc fragment is fused to the C-terminus of a single-domain antibody, and three PAC1 ECDs of the three peptide chains are connected to each other via a linker.

In some embodiments, the above-mentioned single-domain antibody is a single-domain antibody that binds to the CGRP receptor.

In some embodiments, the above-mentioned single-domain antibody comprises a CDR1 as shown in the amino acid sequence of SEQ ID NO: 2, 7, 12, 17, 22, 27, 32, 37, 42, 47, 52, or 57 or any variant thereof, a CDR2 as shown in the amino acid sequence of SEQ ID NO: 3, 8, 13, 18, 23, 28, 33, 38, 43, 48, 53, or 58 or any variant thereof, and a CDR3 as shown in the amino acid sequence of SEQ ID NO: 4, 9, 14, 19, 24, 29, 34, 39, 44, 49, 54, or 59 or any variant thereof.

In some embodiments, the above-mentioned single-domain antibody comprises CDR1 as shown in the amino acid sequence of SEQ ID NO: 12 or any variant thereof, CDR2 as shown in the amino acid sequence of SEQ ID NO: 13 or any variant thereof, and CDR3 as shown in the amino acid sequence of SEQ ID NO: 14 or any variant thereof.

In some preferred embodiments, the amino acids in one or more or all three of the above-mentioned CDRs have conservative substitutions, and the number of conservatively substituted amino acids does not exceed 1, 2, 3, 4, 5, 6, 7, 8, 9, or 10.

In some embodiments, the single-domain antibody comprises a variable region as shown in the amino acid sequence of SEQ ID NO: 1, 6, 11, 16, 21, 26, 31, 36, 41, 46, 51, 56, 61, 62, or 63 or any variant thereof.

In some preferred embodiments, the single-domain antibody comprises a variable region as shown in the amino acid sequence of SEQ ID NO: 61, 62, or 63 or any variant thereof.

In some more preferred embodiments, the single-domain antibody comprises a variable region as shown in the amino acid sequence of SEQ ID NO: 61 or any variant thereof.

In some preferred embodiments, the amino acids in the above-mentioned single-domain antibodies have conservative substitutions, and the number of conservatively substituted amino acids does not exceed 1, 2, 3, 4, 5, 6, 7, 8, 9, or 10.

In some embodiments, the PAC1 ECD is selected from the ECD of PAC isomers: PAC1, PAC1s, or PAC1vs.

In some preferred embodiments, the PAC1 ECD is PAC1s ECD.

In some preferred embodiments, the PAC1 ECD comprises the amino acid sequence of SEQ ID NO: 64 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto.

In some preferred embodiments, the PAC1s ECD comprises the amino acid sequence of SEQ ID NO: 66 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto.

In some embodiments, the Fc fragment comprises a hinge region, a constant region 2 (CH2), and a constant region 3 (CH3) domain derived from a human immunoglobulin.

In some preferred embodiments, the Fc fragment is selected from human IgG1, IgG2, IgG3, and IgG4; more preferably, the Fc fragment is selected to be human IgG1.

In some preferred embodiments, the Fc fragment has an amino acid substitution; more preferably, the amino acid substitution includes replacement of asparagine N at position 297 on the Fc fragment by alanine A, wherein each amino acid site on the Fc fragment is numbered using the EU antibody numbering system.

In some preferred embodiments, the Fc fragment comprises the amino acid sequence of SEQ ID NO: 68 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto.

In some embodiments, the linker is a flexible polypeptide linker.

In some preferred embodiments, the above-mentioned flexible polypeptide linker is selected from GGGGS, GGGGSGGGGS, SGGGGSGGGG, GGGGSGGGGSSGGGGS, GGGGSGGGGSGGGGS, GGGGSGGGGSGGGG, GGGGSGGGGSGGGGSGGGGS, GGGGSGGGGSGGGSGGGG, GSGSGSGS, GGSGSGSG, GGSGSG, and GGSG.

In some more preferred embodiments, the flexible polypeptide linker is GGGGS (SEQ ID NO: 79).

In another aspect, the present invention provides a nucleic acid encoding the above-mentioned fusion protein.

In another aspect, the present invention provides a vector comprising the above nucleic acids.

In another aspect, the present invention provides cells containing the nucleic acids or vector.

In another aspect, the present invention provides a pharmaceutical composition or kit, which comprises the above-mentioned fusion protein and a pharmaceutically acceptable vector, diluent, or excipient.

In another aspect, the present invention provides a use of the above-mentioned fusion protein, nucleic acid, vector, and/or cells and/or pharmaceutical composition or kit in the preparation of a drug for treating diseases related to CGRP and PACAP pathway activation.

In some preferred embodiments, the above disease is migraine.

The above description is only preferred embodiments of the present invention and an illustration of the technical principles used. Those skilled in the art should understand that the scope of disclosure involved in the present invention is not limited to the technical solutions formed by a specific combination of the above-mentioned technical features, but should also cover other technical solutions formed by any combination of the above-mentioned technical features or their equivalent features without departing from the concepts disclosed above, for example, a technical solution formed by replacing the above-mentioned features with (but not limited to) technical features with similar functions disclosed in the present invention.

**Examples**

**Example** 1: **Synthesis of single-domain antibodies against CGRP receptor**

By immunizing llamas with the ECD of the CGRP receptor, the optimal sequence 42hv10 of the single-domain antibody against the CGRP receptor was obtained after multiple rounds of screening and humanization.

In order to obtain highly selective and high-affinity binding antibodies, a protein fused with in vitro expressed and purified CGRP ECD and the crystallizable fragment (Fc) of human immunoglobulin G1 (IgG1) (referred to as RC-Fc) was used as an immunogen to immunize llamas, then lymphocytes were separated from high-titer llama blood, and their messenger ribonucleic acid (mRNA) was extracted. These mRNAs were used to construct a phage library, and then the enzyme-linked immunosorbent assay (ELISA) was used to screen out positive colonies containing single-domain antibodies that could bind to RC-Fc and positive colonies containing single-domain antibodies that could only bind to Fc. These colonies included all single-domain antibodies that bind to CGRP ECD and Fc, colonies that only bind to Fc were excluded, and the remaining CGRP ECD-positive and Fc-negative colonies were sent for sequencing. Through sequence analysis and verification, a batch of single-domain antibodies that can specifically bind to CGRP ECD were obtained.

1. Preparation of RC-Fc

1.1 Design of RC-Fc

RC-Fc is formed through the fusion of CGRP receptor: human Ramp1 ECD, CLR ECD, and Fc. The Ramp1 ECD is from Genbank ID NM_005855.4 nucleic acid sequence 132-404 (SEQ ID NO: 69). The nucleic acid sequence is as follows:

```
tgccaggaggctaactacggtgccctcctccgggagctctgcctcacccagttccaggtagacatggaggccgtcgggggagacgctgtggtgtgactggggcagg
accatcaggagctacaggggagctggccgactgcacctggcacatggcggagaagctgggctgcttctggcccaatgcagaggtggacaggttcttcctggcagtg
catggccgctacttcaggagctgccccatctcaggcagggccgtgccgggacccgcccggcagc
```

the corresponding amino acid sequence is from NP_005846.1 27-117 (SEQ ID NO: 70). The amino acid sequence is as follows:

```
CQEANYGALLRELCLTQFQVDMEAVGETLWCDWGRTIRSYRELADCTWHMAEKLGCFWPNAEVDRFF
LAVHGRYFRSCPISGRAVRDPPGS
```

the CLR ECD is from Genbank ID NM_001271751.2 nucleic acid sequence 407-782 (SEQ ID NO: 71). The nucleic acid sequence is as follows:

```
agaattagaagagagtcctgaggactcaattcagttgggagttactagaaataaaatcatgacagctcaatatgaatgttaccaaaagattatgcaagacccccattcaac
aagcagaaggcgtttactgcaacagaacctgggatggatggctctgctggaacgatgttgcagcaggaactgaatcaatgcagctctgccctgattactttcaggactt
tgatccatcagaaaaagttacaaagatctgtgaccaagatggaaactggtttagacatccagcaagcaacagaacatggacaaattatacccagtgtaatgttaacacc
cacgagaaagtgaagactgcactaaatttgttttacctgaccataat
```

the corresponding amino acid sequence is from NP_001258680.1 amino acid sequence 23-146 (SEQ ID NO: 72). The amino acid sequence is as follows:

```
ELEESPEDSIQLGVTRNKIMTAQYECYQKIMQDPIQQAEGVYCNRTWDGWLCWNDVAAGTESMQLCPD
YFQDFDPSEKVTKICDQDGNWFRHPASNRTWTNYTQCNVNTHEKVKTALNLFYLTI
```

the Fc portion is from Genbank ID JX292763.1 nucleic acid sequence 661-1359 (SEQ ID NO: 73). The nucleic acid sequence for cloning is as follows:

```
gagcccaaatcttgtgacaaaactcacacatgcccaccgtgcccagcacctgaactcctggggggaccgtcagtcttcctcttccccccaaaacccaaggacaccct
catgatctcccggacccctgaggtcacatgcgtggtggtggacgtgagccacgaagaccctgaggtcaagttcaactggtacgtggacggcgtggaggtgcataat
gccaagacaaagccgcgggaggagcagtacaacagcacgtaccgggtggtcagcgtcctcaccgtcctgcaccaggactggctgaatggcaaggagtacaagt
gcaaggtctccaacaaagccctcccagcccccatcgagaaaaccatctccaaagccaaagggcagccccgagaaccacaggtgtacaccctgcccccatcccgg
gatgagctgaccaagaaccaggtcagcctgacctgcctggtcaaaggcttctatcccagcgacatcgccgtggagtgggagagcaatgggcagccggagaacaa
ctacaagaccacgcctcccgtgctggactccgacggctccttcttcctctacagcaagctcaccgtggacaagagcaggtggcagcaggggaacgtcttctcatgct
ccgtgatgcatgaggctctgcacaaccactacacgcagaagagcctctccctgtctccgggtaaatga
```

the corresponding amino acid sequence Genbank ID AFR78282.1 221-452 (SEQ ID NO: 74) is as follows:

```
EPKSCDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKSRWQQGNVF
SCSVMHEALHNHYTQKSLSLSPGK
```

Alternatively, the Fc portion is from Genbank ID MW176619.1 nucleic acid sequence 663-1358 (SEQ ID NO: 91). The nucleic acid sequence for cloning is as follows:

```
gagcccaaatcttgtgacaaaactcacacatgcccaccgtgcccagcacctgaactcctggggggaccgtcagtcttcctcttccccccaaaacccaaggacaccct
catgatctcccggacccctgaggtcacatgcgtggtggtggacgtgagccacgaagaccctgaggtcaagttcaactggtacgtggacggcgtggaggtgcataat
gccaagacaaagccgcgggaggagcagtacaacagcacgtaccgtgtggtcagcgtcctcaccgtcctgcaccaggactggctgaatggcaaggagtacaagtg
caaggtctccaacaaagccctcccagcccccatcgagaaaaccatctccaaagccaaaggggcagccccgagaaccacaggtgtacaccctgcccccatcccggg
aggagatgaccaagaaccaggtcagcctgacctgcctggtcaaaggcttctatcccagcgacatcgccgtggagtgggagagcaatgggcagccggagaacaac
tacaagaccacgcctcccgtgctggactccgacggctccttcttcctctatagcaagctcaccgtggacaagagcaggtggcagcaggggaacgtcttctcatgctc
cgtgatgcatgaggctctgcacaaccactacacgcagaagagcctctccctgtccccgggtaaa
```

the corresponding amino acid sequence Genbank ID QRG33954 221-452 (SEQ ID NO: 92) is as follows:

```
EPKSCDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDKSRWQQGNVF
SCSVMHEALHNHYTQKSLSLSPGK
```

Due to the different amino acid series of Ramp1 ECD and CLR ECD, they need to be paired through the Knob in Hole (KiH) technique when fused with Fc, and the C-terminus of Ramp1 ECD is connected to the Fc N-terminus with T366W mutation to form the R chain; the C-terminus of CLR ECD is fused to the Fc N-terminus with T366S, L368A, and Y407V mutations to form the C chain.

The amino acid sequence of the RC-Fc Ramp1 ECD (RC-Fc R) chain (SEQ ID NO: 75) is as follows:

```
CQEANYGALLRELCLTQFQVDMEAVGETLWCDWGRTIRSYRELADCTWHMAEKLGCFWPNAEVDRFF
LAVHGRYFRSCPISGRAVRDPPGSEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKEYKCKVSNKALP
APIEKTISKAKGQPREPQVYTLPPSREEMTKNQVSLWCLVKGFYPSDIAVEWESNGQPENNYKTTPPVLD
SDGSFFLYSKLTVDKSRWQQGNVFSCSVMHEALHNHYTQKSLSLSPGK
```

wherein the Fc fused to the RC-Fc R chain has a T366W mutation, as shown below (SEQ ID NO: 76):

```
EPKSCDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPS
REEMTKNQVSLWCLVKGFYPSDIAVEWESNGQPENNYKTTPPVLDSDGSFFLYSKLTVDKSRWQQGNV
FSCSVMHEALHNHYTQKSLSLSPGK
```

The amino acid sequence of the RC-Fc CLR ECD (RC-Fc C) chain (SEQ ID NO: 77) is as follows:

```
ELEESPEDSIQLGVTRNKIMTAQYECYQKIMQDPIQQAEGVYCNRTWDGWLCWNDVAAGTESMQLCPD
YFQDFDPSEKVTKICDQDGNWFRHPASNRTWTNYTQCNVNTHEKVKTALNLFYLTIEPKSCDKTHTCPP
CPAPELLGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREEMTKNQVSLS
CAVKGFYPSDIAVEWESNGQPENNYKTTPPVLDSDGSFFLVSKLTVDKSRWQQGNVFSCSVMHEALHN
HYTQKSLSLSPGK
```

wherein the Fc fused to the RC-Fc C chain has T366S, L368A, and Y407V mutations as shown below (SEQ ID NO: 78):

```
EPKSCDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPS
REEMTKNQVSLSCAVKGFYPSDIAVEWESNGQPENNYKTTPPVLDSDGSFFLVSKLTVDKSRWQQGNV
FSCSVMHEALHNHYTQKSLSLSPGK
```

### 1.2 Gene synthesis, expression, and purification of RC-Fc

Taizhou Biotechnology Co., Ltd. (Biointron) was commissioned to synthesize the RC-Fc plasmid, and the method was as follows: the synthesized gene fragment was ligated with the pcDNA3.4 vector (ThermoFisher Scientific, EL0011) after NotI (New England BioLabs Inc., NEB, R0189L)/XbaI (New England BioLabs Inc., NEB, R0145L) digestion and recovery (made by Biointron). The ligation product was transformed into Top10 competent cells, positive clones were picked for scale-up culture, and a small amount of plasmid was extracted and sequenced using the plasmid mini-extraction kit prepared by Biointron (made by Biointron). The clones with correct sequencing were inoculated into an LB medium (made by Biointron) for scale-up culture, and the plasmids were extracted in large quantities using a kit prepared by Biointron (made by Biointron) and sequenced again for verification.

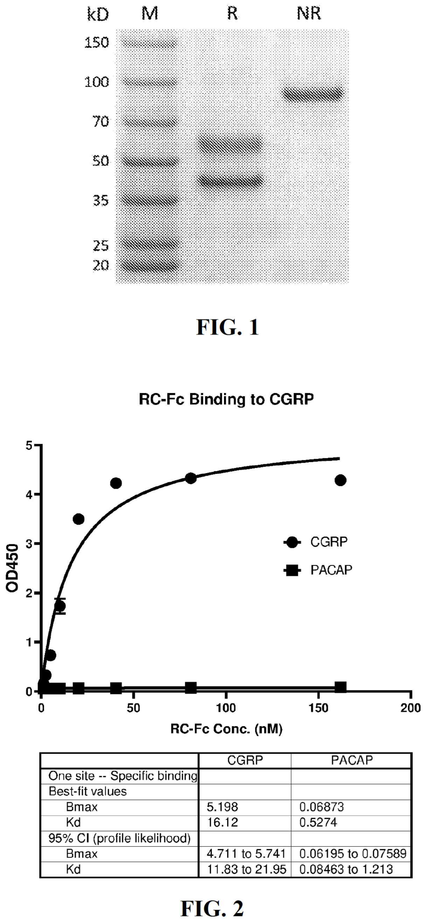

The expression plasmid was transfected into 293 cells using a transfection reagent (made by Biointron), and the cell culture was harvested after culturing in a constant temperature incubator (Crystal, IS-RDS6C5) for 5 days. The cell culture was centrifuged, the supernatant was taken, the cell debris was removed by filtration, and the clarified fluid was collected. The target protein was captured using a Protein A affinity chromatography column. The target protein was filtered, the final yield was determined by measuring A280, and the absorbance at 280 nm was read using a NanoDrop One (ThermoFisher Scientific, ND-ONE-W) instrument. The protein was transferred to a dialysis bag and dialyzed in a beaker containing 1X PBS. The purified product was identified by means of electrophoresis, and the electrophoresis results are shown in FIG. 1.

### 2. Verification of RC-Fc binding to CGRP

The expressed and purified RC-Fc fusion protein was used for animal immunization only after being verified to be able to bind to its ligand CGRP. The verification was done by the enzyme-linked immunosorbent assay (ELISA) of Chengdu NB Biolab Co., Ltd. specifically as follows.

A streptavidin-coated 96-well ELISA plate (ThermoFisher Scientific™, 15126) was washed three times with PBST (1xPBS (Absin, abs9266), 0.1% Tween 20 (Solarbio, T8220), pH=7.4), and then 1000 ng of Biotin-CGRP (Nanjing Yuan-Peptide Biotechnology Co., Ltd., A03Biotin137, P200629-HS319172) was added to each well and incubated at room temperature for 2 hours. PBST was removed, and 100 μL of RC-Fc fusion protein serially diluted with PBST was added to each well and incubated at 37°C for 1 hour. The ELISA plate incubated with the samples was washed five times with PBST, then 100 μL of the corresponding secondary antibody Anti-human IgG-HRP (anti-human immunoglobulin G-horseradish peroxidase, Hangzhou HuaBio, HR1214) diluted 1:5000 with a blocking solution (PBST + 3% skim milk powder (Solarbio, D8340)) was added, and incubated at 37°C for 1 hour.

The ELISA plate incubated with the secondary antibody was washed five times with PBST, 100 μL of TMB (Trimethylolpropane) single-component colorimetric solution (Solarbio, 20190402) was added to each well and incubated at 37°C for 7 min, 100 μL of 1M HCL (Kelong Chemicals) was added to each well to terminate the reaction, and the OD value was read at a wavelength of 450 nm using an ELISA reader (ThermoFisher Scientific, 51119080ET). The ODOD450 value was analyzed using the Nonlinear Regression One Site Specific Binding mode of GraphPad Prism9.3.1, and it was obtained that the Bmax of RC-Fc binding to CGRP was 5.198 and Kd was 16.12 nM. The Bmax of RC-Fc binding to the negative control polypeptide PACAP was 0.069, and the Kd was invalid. The results are shown in FIG. 2, indicating that the reconstructed CGRP receptor extracellular region of the present invention retains the ability to bind to its ligand CGRP but does not bind to irrelevant polypeptide PACAP.

### 3. Immunization of llama with RC-Fc

The llama immunization and VHH screening process were entrusted to Chengdu NB Biolab Co., Ltd. (NB Biolab). Before immunization, 10 mL of blood was collected as a negative serum control, and then 0.5 mg RC-Fc was mixed homogeneously with 1 mL of Complete Freund's Adjuvant (CFA, Sigma, F5881), which was injected subcutaneously. On day 21, 0.25 mg of the antigen was mixed homogeneously with 1 mL of Incomplete Freund's Adjuvant (IFA, Sigma, F5506) and then injected subcutaneously. On day 28, 10 mL of blood was collected to separate the serum. On day 42, 0.25 mg of antigen was mixed homogeneously with 1 mL of IFA and injected subcutaneously. On day 49, 50 mL of peripheral blood was collected to separate lymphocytes and serum. On day 63, 0.25 mg of the antigen was mixed homogeneously with 1 mL of IFA and injected subcutaneously. On day 70, 50 mL of peripheral blood was collected to separate lymphocytes and serum.

### 4. Gradient Detection

RC-Fc was diluted to 2000 ng/mL with a coating solution (50 mM $NaHCO_3$ PH=9.6), and 100 $\mu$L was added to each well of a 96-well ELISA plate, which was left at 4°C overnight. The ELISA plate coated overnight at 4°C was washed five times with PBST, patted dry, and 300 $\mu$L of a blocking solution was added to each well.

The serum was serially diluted 1:2 with TBST, the first gradient serum was 1:2000, and the last gradient serum was 1:128000. After the antigen plate was washed three times with 300 $\mu$L/well PBST, 100 $\mu$L of diluted serum was added to each well, incubated at 37°C for 1 hour, washed three times with 300 $\mu$L/well PBST, 100 $\mu$L of 1:2000 anti-M13 antibody (NB Biolab, 052-101-005) was added to each well, incubated at 37°C for 1 hour, washed six times with 300 $\mu$L/well PBST, and 1:10000 diluted anti-Alpaca-HRP (NEB, S001H) was added, at 37°C for 30 minutes. The ELISA plate incubated with the secondary antibody was washed 5 times with PBST, 100 $\mu$L of TMB single-component colorimetric solution was added to each well, incubated at 37°C for 7 minutes, 100 $\mu$L of 1M HCL was added to each well to terminate the reaction, and the OD450 reading was carried out.

### 5. Construction of phage library

High-gradient peripheral blood was selected, and PBMC was separated according to the instructions of lymphocyte separation medium (TBD sciences, LTS1077). Total RNA was extracted using RNAiso Plus (Foregene, RE-03111) reagent, and 5 $\mu$g of RNA was transcribed into cDNA using PrimeScript™ II 1st Strand cDNA Synthesis Kit (Takara, 9109). The cDNA stock solution was diluted x5 for nested PCR amplification, and the single-domain antibody (VHH) fragment of about 750 base pairs (BP) was recovered by cutting the gel for a second round of PCR amplification. The purified PCR amplification product was the target VHH fragment. The target VHH fragment was connected to the vector pComb3XSS (NEB, P001) through the Sfil restriction site. Immediately after electroporation, 1 mL of 2YT (a culture medium containing tryptone (Solarbio, T8490), yeast extract (Solarbio, Y8020), and sodium chloride (Kelong Chemicals), preheated at 37°C) was added to the electroporation cup for recovery. The electroporation product was aspirated, and the electroporation cup was washed with 2YT culture medium. A total of 100 mL of a recovery product was obtained, recovered at 37°C and 180 rpm for 45 minutes, 100 $\mu$L was taken for gradient dilution to $10^{-3}$ and $10^{-4}$ to determine the number of library transformants, which was spread on a 90 mm plate, and the rest was centrifuged, resuspended in 8 mL of 2YT, and spread on 8 200 mm plates. On the next day, bacterial clones carrying the target VHH fragment were obtained on plates for determining the number of library transformants.

### 6. Phage library packaging

The bacterial library was inoculated into 2 × 300 mL 2YT+A (100 $\mu$g/mL of Ampicillin (Solarbio, A1170)) + G (1% glucose (Solarbio, G8150)) medium until its initial OD600 = 0.1-0.2, and cultured at 37°C and 230 rpm until OD600 = above 0.8. Helper phage M13KO7 (NBbiolab, P006) and TG1 bacteria (NBbiolab, P008) were added according to the OD600 value (helper phage: bacteria = 20:1). After adding M13KO7, mixed homogeneously, left undisturbed at 37°C for 30 minutes, shook slowly at 180 rpm for 30 minutes, centrifuged at 5000 rpm for 10 minutes, the supernatant was discarded, the precipitate was resuspended with an equal volume of 2YT+A+K (kanamycin (Solarbio, K1030) 50 $\mu$g/ml) culture medium overnight at 30°C and 220 rpm.

The overnight culture was centrifuged at 4°C, 10000 rpm for 20 minutes, the supernatant was collected, and the precipitate was discarded. The centrifuge tube was replaced, centrifuged again at 4°C, 10000 rpm for 20 min, and the supernatant was collected.

PEG8000 (Solarbio, P8260)/NaCl was added at 1/5 of the supernatant volume, mixed homogeneously, and precipitated on ice for more than 2 hours. Centrifuged at 10000 rpm for 20 minutes, the supernatant was discarded, and all the supernatant was removed after one centrifuge. The precipitate was resuspended in 1 mL 1× PBS, and 1/5 volume of PEG8000/NaCl was added for secondary precipitation for 1 hour.

Centrifuged at 12000 rpm for 10 minutes, the supernatant was discarded, and all the supernatant was removed after one centrifuge. According to the amount of the precipitate, 1× PBS was added to resuspend the precipitate, and 100% glycerol was added to a final concentration of 50%, which was mixed homogeneously and dispensed into 1.5 mL EP (Eppendorf) tubes and stored at -80°C.

### 7. Screening of positive colonies binding to RC-Fc (positive screening)

RC-Fc was diluted to a final concentration of 5 $\mu$g/mL using a coating solution with a pH of 9.6, added to the enzyme-labeled wells at 100 μL/well, eight wells were coated for each target molecule (four wells for the second round of screening and two wells for the third round of screening), and the coating was carried out at 4°C overnight. The coating solution was then discarded, the cells were washed three times with PBS, 300 μL of 3% BSA-PBS blocking solution was added to each well, and the cells were blocked at 37°C for 1 hour. Then, washed three times with PBS, 100 μL of the phage library was added, and incubated at 37°C for 1 hour. The unbound phages was aspirated, washed with PBST 6 times, washed with PBS 2 times, 100 μL Gly-HCl eluent was added, incubated at 37°C for 8 minutes, and the specifically bound phages was eluted; the eluate was transferred to a 1.5 mL sterile centrifuge tube, quickly neutralized with 10 μL Tris-HCl neutralization buffer, 10 μL was taken for gradient dilution, the titer was determined, and the panning recovery rate was calculated.

24 single clones were randomly picked from the eluate titer plate with a sterilized toothpick, inoculated into 1 mL 2×YT-A, and shaken and cultured at 37°C and 220 rpm for 8 hours. 200 μL of the above culture was taken, M13K07 phage was added at a ratio of phage:TG1 = 20:1, which was left undisturbed at 37°C for 15 minutes, and then shaken and cultured at 220 rpm for 45 minutes. 800 μL of 2×YT-AK was added, and cultured at 30°C with vigorous shaking overnight. On the next day, the cells were centrifuged at 12000 rpm for 2 minutes, and the supernatant was taken for monoclonal ELISA identification.

8. Screening of positive clones binding to Fc (negative screening)

The Fc protein (NBbiolab, 20200706) was diluted with a coating solution with a pH of 9.6 to a final concentration of 2 μg/mL, and 100 μL/well was added to the ELISA wells for coating overnight at 4°C. Then, the coating solution was discarded, and the wells were washed 3 times with PBST. 200 μL of 5% skim milk was added to each well, and the wells were blocked at 37°C for 1 hour. Then, the wells were washed 3 times with PBST, and 50 μL of phage culture supernatant and 50 μL of 5% skim milk were added to each well, and incubated at 37°C for 1 hour. After washing with PBST for 6 times, horseradish peroxidase-labeled anti-M13 antibody (diluted 1:10000 in PBS) was added at 100 μL/well for 1 hour of action at 37°C. The plate was washed 6 times with PBST. TMB color development solution was added for color development, 100 μL/well, 37°C, 7 minutes. A stopping solution was added to terminate the reaction, 50 μL/well, and the optical density was measured at 450 nm.

9. Exclude Fc-binding positive clones

The results of positive screening and negative screening were analyzed comprehensively, with the goal of retaining positive clones from the positive screening and excluding positive clones from the negative screening. Finally, the positive clones from the positive screening and negative clones from the negative screening were sent to Chengdu Tsingke Biotech Co., Ltd. for sequencing.

10. Results Sequence Analysis

Three llamas were immunized with RC-Fc, and the peripheral blood lymphocyte mRNAs of two of the animals were used to establish a phage library. After screening and sequencing, a total of 567 VHH sequences were obtained (311 from animal NB268 and 258 from animal NB269). After sequence comparison, 74 sequences (16 in NB268 and 58 in NB269) were determined to be unique. Finally, 12 VHH sequences with relatively high bacterial culture OD450 values and no obvious hot spot amino acids (such as extra cystine, N-linked glycosylation sites) were selected from these 74 unique VHH sequences for cloning, expression, and purification. The sequences of these 12 VHHs are as follows:

A10-268-5:

Nucleotide sequence (SEQ ID NO: 5)

caggtgcagctcgtggagtcggggcggaggattggtgcaggctggggggctctctgagactctcctgtgcagtctctggacgcacagtcactaccattaccatgggctgg ttccgccaggctccagggaaggagcgtgaatttgtagcaagtattagctgggatggtcttagcacaggctatcaagactccgtgaagggccgattcaccatctccagag acaacgccaagaacacggtgtatctgcaaatgaacagcctgaaacctgaggacacggccgtttattactgtgcagcgggcgacagtttatggtcatggtcgaaggttc cagctaattatgcctactggggccaggggacccaggtcaccgtctcctca

Amino acid sequence (SEQ ID NO: 1)

```
<----------------FR1-IMGT---------------><---CDR1--><---------FR2-IMGT---------><--CDR2---><-------------------
--
QVQLVESGGGLVQAGGSLRLSCAVSGRTVTTITMGWFRQAPGKEREFVASISWDGLSTGYQDSVKGRF
TI

-----FR3--IMGT----------------------------->----CDR3---->-------------FR4-IMGT--------------->
SRDNAKNTVYLQMNSLKPEDTAVYYCAAGDSLWSWSKVPANYAYWGQGTQVTVSS
```

A10-268-18:

Nucleotide sequence (SEQ ID NO: 10)

caggtgcagctcgtggagtcgggggggaggccaggtacaggctggggggctctctgagactctcctgtgcagcctctggacgcataagtagtagctatgacatgggctg
gctccgccaggctccagggaaggagcgtgagtttgtagcacggattacctggagtggtggtagcacatactatgcagactccgtgaaggggccgattcaccatctccag
agacagcaccaagaagacggtgtatctgcaaatgaacagcctgaaacctgaggacacggccgtttattactgtgcagcaaaccgagcctttactggtcagtatgactac
tggggccaggggacccaggtcaccgtctcctca

Amino acid sequence (SEQ ID NO: 6)

```
<----------------FR1-IMGT---------------><--CDR1--><-------FR2-IMGT----------><---CDR2-I--><-------------------
--
QVQLVESGGGQVQAGGSLRLSCAASGRISSSYDMGWLRQAPGKEREFVARITWSGGSTYYADSVKGRF
TI

----FR3-IMGT-------------------------------><-------CDR3--------><---FR4-IMGT-->
SRDSTKKTVYLQMNSLKPEDTAVYYCAANRAFTGQYDYWGQGTQVTVSS
```

A10-268-42:

Nucleotide sequence (SEQ ID NO: 15)

caggtgcagctcgtggagtctggaggaggattggtgcaggctggggactctctgagactctcctgtgcagcctctggacgcaccttcagtggatatgtcatgggctggt
tccgccaggctccagggaaggagcgtgagtttgtagcagctcttaggtggagtagtagtaacacaaactatgcggcgtccgtgaaggggccgattcaccatctccagag
actacgccaaggacacggtgtatctgcaaatgaacagcctcaaacctgacgacacggccgtttattactgtgcagcgaaacgggatcggccgtacggtagtacgtgg
accccagttgcagctgactatgacacttggggccaggggacccaggtcaccgtctcctca

Amino acid sequence (SEQ ID NO: 11)

```
<---------------FR1-IMGT------------------><---CDR1---><---------FR2-IMGT---------><--CDR2---><-----------------
-
QVQLVESGGGLVQAGDSLRLSCAASGRTFSGYVMGWFRQAPGKEREFVAALRWSSSNTNYAASVKGR
F

---------FR3-IMGT-------------------------------><-----------------CDR3----------------><---FR4-IMGT--->
TISRDYAKDTVYLQMNSLKPDDTAVYYCAAKRDRPYGSTWTPVAADYDTWGQGTQVTVSS
```

A10-268-76:

Nucleotide sequence (SEQ ID NO: 20)

caggtgcagctcgtggagtctggggggaggattggtgcctgctggggggctctctgaacctctcctgtgcagcctctggacgcaccttcagtacctatgccatgggctggt
tccgccaggctccagggaaggagcgtgagtttgtcgcgactattaactgggataatggtcgcacatattatgcagactccgtgaagggggccgattcaccatctccagaga
caacgcaaagaacatggtgtatctacaaatgaacagcctgaaacctgaggacacggccgtttattactgtgcagcagataagacggccatatttcgtacgagtgttgtgc
cagatgactatgactactggggccaggggacccaggtcaccgtctcctca

Amino acid sequence (SEQ ID NO: 16)

```
<--------------FR1-IMGT----------------><---CDR1---><---------FR2-IMGT--------><---CDR2---><-----------------
--
QVQLVESGGGLVPAGGSLNLSCAASGRTFSTYAMGWFRQAPGKEREFVATINWDNGRTYYADSVKGR
FT
-------------FR3-IMGT-------------------------------------------------><----CDR3---><---FR4-IMGT-->
ISRDNAKNMVYLQMNSLKPEDTAVYYCAADKTAIFRTSVVPDDYDYWGQGTQVTVSS
```

A10-268-103:

Nucleotide sequence (SEQ ID NO: 25)

caggtgcagctcgtggagtctggaggaggattggtgcaggctggggggctctctgagactctcctgtgtagcgtctggacgcacttcagtagctatgccatgggctggt
tccgccaggctccagggaaggagcgtgagtttgtgtcaggtattaccgagagtggtgctaccacacgctatgcagactccatgaggggccgattcatcatctccagag
acaacgccagaacacggtatatctggaaatgaacagcctgaaacctgaggactcggccgtttattactgtgcagcgagtcggtcggattactgtaccgactgctaa
agggggcctataactactggggccaggggacccaggtcaccgtctcctca

Amino acid sequence (SEQ ID NO: 21)

```
<----------------FR1-IMGT----------------><---CDR1---><--------FR2-IMGT-------><---CDR2---><--------------------
-
QVQLVESGGGLVQAGGSLRLSCVASGRTFSSYAMGWFRQAPGKEREFVSGITESGATTRYADSMRGRFI
I
-----------FR3-IMGT-----------------------------------------><----CDR3---><---FR4-IMGT-->
SRDNAQNTVYLEMNSLKPEDSAVYYCAASRFGITVPTAKGAYNYWGQGTQVTVSS
```

A10-268-273:

Nucleotide sequence (SEQ ID NO: 30)

gaggtgcagctggtggagtccggaggaggattggtgcagccgggggactctctcacacttcctgcgcagcctctggacgcgccaagaatcgctatgtcatggcctg
gttccgccaggctccagggaaggagcgtgagtttgtagcagctcttaggtggagtagtagtaacacaaactatgcggccgtccgtgaagggccgattcaccatctccag
agactacgccaaggacacggtgtatctgcaaatgaacagcctcaaacctgacgacacggccgtttattactgtgcagcgaaacgggatcggccgtacggtagtacgt
ggaccccagttgcagctgactatgacacttggggccaggggacccaggtcaccgtctccgca

Amino acid sequence (SEQ ID NO: 26)

```
<---------------FR1-IMGT---------------><----CDR1----><--------FR2-IMGT--------><----CDR2---><-----------------
-
EVQLVESGGGLVQPGDSLTLSCAASGRAKNRYVMGWFRQAPGKEREFVAALRWSSSNTNYAASVKGR
F
-----------FR3-IMGT-----------------------------------------><-------CDR3-------><---FR4-IMGT--->
TISRDYAKDTVYLQMNSLKPDDTAVYYCAAKRDRPYGSTWTPVAADYDTWGQGTQVTVSA
```

A10-269-56:

Nucleotide sequence (SEQ ID NO: 35)

gaggtgcagctggtggagtcggggggaggcttggtgcaggctggggggtctctgagactctcctgtgcagcctctggaagcacttcagtggccgttacatgggctg
gttccgtcagggtcaagggaaggagcgtgagtttgtagcagcttcaatgtggagtggtactgcaatatcgtacgcagactccgtgaagggccgattcaccatctccaga
gacaacgccaaggccacgatgtatctgcaaatgagcagcctgaaagatgaggacacggccgtttattactgcgcagcaggaggattgtggaatctcaagctttcagac
gcagggtattatgaatactggggccaggggacccaggtcaccgtctcctca

Amino acid sequence (SEQ ID NO: 31)

```
<----------------FR1-IMGT----------------><---CDR1--><----------FR2-IMGT--------><---CDR2---><----------------
--
```

EVQLVESGGGLVQAGGSLRLSCAAS**GSTFSGRY**MGWFRQGQGKEREFVAA**SMWSGTAI**SYADSVKGR
FT

```
---------------FR3-IMGT----------------------------------------------><---CDR3---><---FR4-IMGT-->
```

ISRDNAKATMYLQMSSLKDEDTAVYYCAAGGLWNLKL**SDAGYYEY**WGQGTQVTVSS

A10-269-62:

Nucleotide sequence (SEQ ID NO: 40)

gaggtgcagctggtggagtccggtggaggcttggtgcagactggggggtctctgagactctcctgtgcagcctctggaagcctcttcagtagcagtgacatggcctgg
taccgccaggttccagggaagcagcgcgagttggtcgcacgtattaattttttggtccacaatttatgcggacgtcgtagagggccgattcaccatctccagacacaa
tgcagagagcacggtgtacctgcaaatgaacagcctgaaagctgaggacactggcgtctattattgtaatatgcggtcacgcattacggaaacggagtactggggcca
ggggacccaggtcaccgtctcctca

Amino acid sequence (SEQ ID NO: 36)

```
<----------------FR1-IMGT----------------><--CDR1--><----------FR2-IMGT---------><-CDR2-><----------------------
--
```

EVQLVESGGGLVQTGGSLRLSCAAS**GSLFSSSD**MAWYRQVPGKQRELVAR**INFFGSTI**YADVVEGRFTIS
R

```
-----FR3-IMGT---------------------------><------CDR3-----><---FR4-IMGT--->
```

HNAESTVYLQMNSLKAEDTGVYYC**NMRSRITETEY**WGQGTQVTVSS

A10-269-165:

Nucleotide sequence (SEQ ID NO: 45)

caggtgcagctcgtggagtcaggtggaggcttggtgcaggctggggggtctctgagactctcctgtgcagcctcaagaacatggttcagtatcggtgccatgggctgg
taccgccaggctccaggggaagcagcgcgagttggtcgcaacaattactagtggtagtagcacgacatatgcagacgccgtgaaggccgattcaccgtctccagag
acaacgccgagaacacggtatatctgcaaatgaacagcctgaaacccgaggacacagccgtctattactgctatacaaagtcccgacgtaagccgtgggacctagag
aatgactactggggccaggggacccaggtcaccgtctcctca

Amino acid sequence (SEQ ID NO: 41)

```
<----------------FR1-IMGT----------------><---CDR1---><---------FR2-IMGT---------><--CDR2--><-------------------
--
```

QVQLVESGGGLVQAGGSLRLSCAAS**RTWFSIGA**MGWYRQAPGKQRELVAT**ITSGSSTT**YADAVKGRFT
V

```
--------FR3-IMGT----------------------------><-----------CDR3----------><---FR4-IMGT--->
```

SRDNAENTVYLQMNSLKPEDTAVYYC**YTKSRRKPWDLENDY**WGQGTQVTVSS

A10-269-172:

Nucleotide sequence (SEQ ID NO: 50)

gaggtgcaggtggtggagtcgggaggaggcttggtgcaggctggggggtctctgagactctcctgtgtatcgtctgaaagcagcttcagtatcaacaccatgcgctgg
taccgccaggctccaggggaaggagcgggaattggtggccgtctattaatagtgctagtagcacatactatggagacgccgtgaaggggccgattcaccatctccagagtt
aacgccaagaacacggtgtatctgcaaatgaacagcctgaaacctgaggacacagccgtctatttctgtaatactcactcagtgttagcgtcaagggattactggggcc
aggggacccaggtcaccgtctcctca

Amino acid sequence (SEQ ID NO: 46)

<------------------FR1-IMGT---------------><--CDR1--><---------FR2-IMGT--------><----CDR2---><---------------------

EVQVVESGGGLVQAGGSLRLSCVSS**ESSFSINT**MRWYRQAPGKERELVAS**INSASSTY**YGDAVKGRFTISR

---FR3-IMGT------------------------------><------CDR3------><---FR4-IMGT-->

VNAKNTVYLQMNSLKPEDTAVYFC**NTIISVLASRDY**WGQGTQVTVSS

A10-269-238:

Nucleotide sequence (SEQ ID NO: 55)

caggtgcagctcgtggagtccggtggaggcttggtgcagcctggggggtctctgagactctcctgtgcagcctctggattcacctcggaaactatgacatgagctgggtccgccaggctccaggaaagggggcccgagtgggtctcaggtattgcgcccgcaggtactatcacacgctatgcagactccgtgaaaggccgattcaccatctccagagacaacgccaagaacacgctgtatctgcaaatgaacagcctgaaacctgaggacacggccgtgtatcgctgtgcaaaaggggctagctggggccaggggacccaggtcaccgtgtcctca

Amino acid sequence (SEQ ID NO: 51)

<-----------------FR1-IMGT----------------><---CDR1---><----------FR2-IMGT--------><--CDR2--><--------------------

QVQLVESGGGLVQPGGSLRLSCAAS**GFTFGNYD**MSWVRQAPGKGPEWVSG**IAPAGTIT**RYADSVKGRFTI

--------FR3-IMGT----------------------------><CDR3><---FR4-IMGT--->

SRDNAKNTLYLQMNSLKPEDTAVYRC**AKGAS**WGQGTQVTVSS

A10-269-385:

Nucleotide sequence (SEQ ID NO: 60)

gaggtgcagctggtggagtccggaggaggcttggtgcaggctggggggtctctgagagtctcctgtcaagcctctgaaatcagtttcagtttccatgccatgggctggtaccgccaggctccagggaagcagcgcgagttggtcgcacgtattactagtattggcaggacaacttatgcagacttcgtgaagggccgattcaccatctccagaggcaacgccaagaacacggtgtatctgcaaatgaacagcctgaaacctgaagacacagccgtctactactgtaatgcagagccaggcgaggtgaattatgagtactggggccaggggacccaggtcaccgtctcctca

Amino acid sequence (SEQ ID NO: 56)

<-----------------FR1-IMGT----------------><--CDR1--><----------FR2-IMGT----------><-CDR2-><---------------------

EVQLVESGGGLVQAGGSLRVSCQAS**EISFSFHA**MGWYRQAPGKQRELVAR**ITSIGRT**TYADFVKGRFTISR

---------FR3-IMGT-----------------------><------CDR3-------><--FR4-IMGT-->

GNAKNTVYLQMNSLKPEDTAVYYC**NAEPGRVNYEY**WGQGTQVTVSS

11. Cloning, expression, and purification of A10-268 and A10-269

A10 is the target code of CGRP receptor, and NB268 and NB269 are the numbers of two llamas. The expression and purification work was carried out by Biointron, and 6 histidines (6xHis) were connected to the C-terminus of the VHH sequence for purification. The synthesized gene fragment was ligated to the pcDNA3.4 vector (ThermoFisher Scientific, EL0011) that was digested and recovered with NotI (NEB, R0189L)/XbaI (NEB, R0145L) (made by Biointron). The ligation product was transformed into Top10 competent cells, positive clones were picked for scale-up culture, and a small amount of plasmid was extracted and sequenced using the plasmid mini-extraction kit prepared by Biointron (made by Biointron). The clones with correct sequencing were inoculated into an LB medium (made by Biointron) for scale-up culture, and the plasmids were extracted in large quantities using a kit prepared by Biointron (made by Biointron) and sequenced again for verification. The expression plasmid was transfected into 293 cells using a transfection reagent (made by Biointron), and the cell culture was harvested after culturing in a constant temperature incubator (Crystal, IS-RDS6C5) for 5 days. The cell culture was centrifuged and the supernatant was taken. The cell debris was filtered out, and the clarified fluid was collected. The target protein was captured using a Ni2 affinity chromatography column. The target protein was filtered, the final yield was determined by measuring A280, and the absorbance at 280 nm was read using a NanoDrop One instrument. The protein was transferred to a dialysis bag and dialyzed in a beaker containing 1X PBS, and the purified product was identified by SDS-PAGE electrophoresis. The expressed 12 proteins are shown in Table 2.

Table 2 Expressed 12 proteins and corresponding concentrations thereof

| # | VHH Name | No. | Concentration (mg/ml) |
|---|---|---|---|
| 1 | A10-268-5 | B828601 | 3.02 |
| 2 | A10-268-18 | B828602 | 3.02 |
| 3 | A10-268-42 | B828603 | 3.02 |
| 4 | A10-268-76 | B828604 | 2.19 |
| 5 | A10-268-103 | B828605 | 2.40 |
| 6 | A10-268-273 | B828606 | 2.15 |
| 7 | A10-269-56 | N220505101 | 2.50 |
| 8 | A10-269-62 | N220505102 | 2.00 |
| 9 | A10-269-165 | N220505103 | 0.40 |
| 10 | A10-269-172 | N220505104 | 1.20 |
| 11 | A10-269-238 | N220505106 | 1.50 |
| 12 | A10-269-385 | N220505107 | 0.90 |

## Example 2: Verification of anti-RC-Fc VHH

1. ELISA verification of anti-RC-Fc VHH

RC-Fc VHH binds to immune antigen

The immune antigen RC-Fc was diluted to 2000 ng mL with a coating solution (50 mM NaHCO3 pH = 9.6), and 100 $\mu$L was used to coat a 96-well ELISA plate (ThermoFisher Scientific, 449824) and incubated at 4°C overnight. The ELISA plate at 4°C overnight was washed five times with PBST, patted dry, and 200 $\mu$L of a blocking solution was added, followed by blocking at 37°C for 2 hours. PBST was removed, and 100 $\mu$L of VHH protein serially diluted with PBST was added to each well and incubated at 37°C for 1 hour. The ELISA plate incubated with the samples was washed five times with PBST, and then 100 $\mu$L of the corresponding secondary antibody Mouse Anti-His-HRP (mouse anti-histidine-peroxidase, Nanjing GenScript, A00186) diluted 1:5000 with a blocking solution was added, and incubated at 37°C for 1 hour.

The ELISA plate incubated with the secondary antibody was washed 5 times with PBST, 100 $\mu$L of TMB single-component colorimetric solution (Solarbio, 20190402) was added to each well, incubated at 37°C for 7 min, 100 $\mu$L of 1M HCL (Kelong Chemicals) was added to each well to terminate the reaction, and finally, the OD450 reading was taken with an ELISA reader. The results were analyzed using the Nonlinear regression log (agonist) vs. response (three parameters) model in the GraphPad Prism 9.3.1 software. The obtained Binding $EC_{50}$ values are shown in Table 3.

Table 3 Expressed 12 proteins and corresponding Binding $EC_{50}$ values thereof

| # | VHH Name | No. | Binding $EC_{50}$ (nM) | | | | |
|---|---|---|---|---|---|---|---|
| | | | Exp1 | Exp2 | Exp3 | Ave | Stdev |
| 1 | A10-268-5 | B828601 | 0.143 | 0.184 | 0.08 | **0.136** | **0.052** |
| 2 | A10-268-18 | B828602 | 0.068 | 0.314 | 0.049 | **0.144** | **0.148** |
| 3 | A10-268-42 | B828603 | 0.025 | 0.15 | 0.021 | **0.065** | **0.073** |
| 4 | A10-268-76 | B828604 | 0.024 | 0.144 | 0.028 | **0.065** | **0.068** |

(continued)

| # | VHH Name | No. | Binding EC$_{50}$ (nM) | | | | |
|---|---|---|---|---|---|---|---|
| | | | Exp1 | Exp2 | Exp3 | Ave | Stdev |
| 5 | A10-268-103 | B828605 | 0.372 | 0.114 | 0.265 | **0.250** | **0.130** |
| 6 | A10-268-273 | B828606 | 0.046 | 0.345 | 0.036 | **0.142** | **0.176** |
| 7 | A10-269-56 | N220505101 | No binding | No binding | No binding | **n/a** | **n/a** |
| 8 | A10-269-62 | N220505102 | 0.035 | 0.030 | 0.028 | **0.031** | **0.004** |
| 9 | A10-269-165 | N220505103 | 0.097 | 0.057 | 0.480 | **0.211** | **0.234** |
| 10 | A10-269-172 | N220505104 | 0.056 | 0.051 | 0.042 | **0.050** | **0.007** |
| 11 | A10-269-238 | N220505106 | 0.091 | 0.073 | 0.081 | **0.082** | **0.009** |
| 12 | A10-269-385 | N220505107 | 0.052 | 0.073 | 0.061 | **0.062** | **0.011** |

2. Cell Verification of Anti-RC-Fc VHH

2.1 SK-N-MC cell line and culture

The primary Neuroblastoma cell line SK-N-MC was from ATCC (HTB-10, lot:70036281). The culture medium was DMEM/F-12 (Gibco, C11330500BT) + 10% FBS, referred to as SK cell culture medium.

2.2 cAMP concentration test of SK-N-MC cells

The concentration of cAMP produced by SK-N-MC cells was tested using the cAMP-GS Dynamic Kit (Cisbio, 62AM4PEB) from Cisbio. Unless otherwise specified, the operation method was carried out according to the instructions of the kit. The test culture medium was DMEM/F1-12+10% FBS+1 mM IBMX (Solarbio, I10010), and the test ELISA plate was a white 384-well plate.

2.2.1 Determination of SK-N-MC cell count and acquisition of CGRP standard curves

On the day of the experiment, cells were separated from the culture flask with 0.25% Trypsin-EDTA (Gibco, 25200-056), washed once with PBS, counted, and diluted to an appropriate concentration with test medium. 10000, 7500, 5000, and 2500 cells per test well were used.

5 μL of the cell solution and 5 μL of CGRP (Nanjing Yuan-Peptide Biotechnology Co., Ltd., catalog number: A03-137, batch number: yuanpeptide-997792) diluted with a stimulation buffer (Stimulation Buffer + 500 μM IBMX) were added to the wells of the ProxiPlate-384 Plus test plate (PerkinElmer, 6008280) in sequence. The final CGRP concentration range was 0 nM or 0.001-50 nM.

After adding CGRP, the test plate was incubated in a 37°C incubator for 15 minutes, and then 5 μL cAMP-d2 and 5 μL Anti-cAMP-Cryptate were added to terminate the reaction. The test plate was left at room temperature for 60 minutes and then read using a fluorescence microplate reader (BMG LabTech, Model: PHERAstar FSX) with a 665/620 filter. The obtained values were used to calculate the ratio using the following formula in Microsoft Excel:

$$Ratio = \frac{Signal\ 665\ nm}{Signal\ 620\ nm} \text{ x } 10000$$

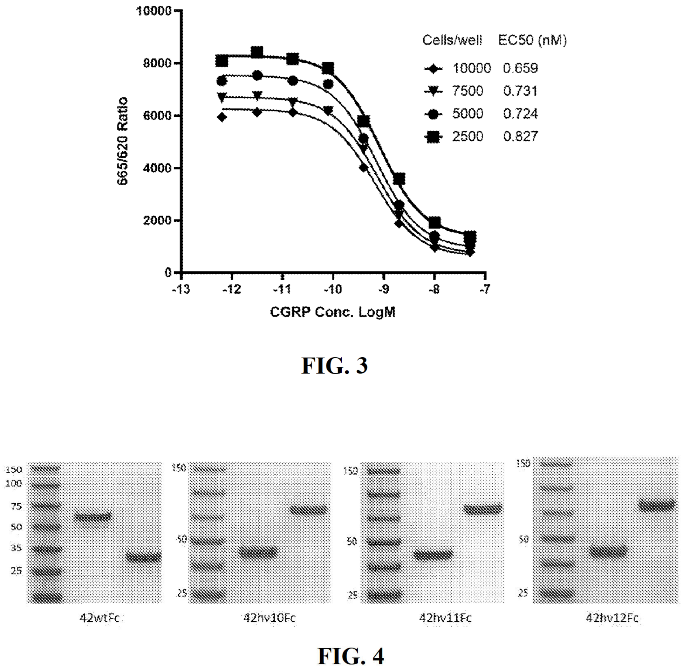

The calculated ratio was analyzed using the Nonlinear regression log (agonist) vs. response (three parameters) model in the GraphPad Prism 9.3.1 software, the results are shown in FIG. 3, indicating that the number of cells per well ranged from 2500 to 10000, and therefore, 2500 cells per well were selected for subsequent experiments.

3. Evaluation of the ability of partially anti-CGRP receptor VHHs to inhibit cellular cAMP production

A10-268-5, A10-268-18, A10-268-42, A10-268-76, A10-268-103, and A10-268-273 were used in cell function tests. On the day of the experiment, SK-N-M cells were separated from the culture flask with 0.25% Trypsin-EDTA, washed once with PBS, counted, and diluted to a cell solution with 5 million cells/mL with a test medium and used within 30 minutes.

5 μL of the cell solution and 5 μL of anti-RC-Fc single-domain antibody diluted 5-fold in series with a stimulation solution (final concentration 0 nM or 0.0001-100 nM) were added to the wells of the test plate, and incubated in a 37°C incubator for 30 minutes.

5 μL of CGRP diluted with a stimulation solution (final concentration is 5 nM) was added, the test plate was placed in a 37°C incubator for 15 minutes of incubation, and then 5 μL of cAMP-d2 and 5 μL of Anti-cAMP-Cryptate were added to terminate the reaction. The test plate was placed at room temperature for 60 minutes and then read using the 665/620 filter of a fluorescent microplate reader, the 665/620 ratio was calculated using the obtained values and then analyzed using the Nonlinear regression log (inhibitor) vs. response (three parameters) model in the GraphPad Prism 9.3.1 software, and the results are shown in Table 4. The $IC_{50}$ values in the table show that four VHHs have the ability to inhibit cAMP production in SK-N-MC cells, and the activity is as follows: A10-268-273 > A10-268-42 > A10-268-103 > A10-268-18. A10-268-42 was selected as the lead VHH for the next step of evaluation.

Table 4 6 VHHs used for cell function tests and their corresponding $IC_{50}$ values

| # | VHH Name | No. | $IC_{50}$ (nM) |
|---|---|---|---|
| 1 | A10-268-5 | B828601 | >10000 |
| 2 | A10-268-18 | B828602 | 492 |
| 3 | A10-268-42 | B828603 | 39.67 |
| 4 | A10-268-76 | B828604 | >10000 |
| 5 | A10-268-103 | B828605 | 279 |
| 6 | A10-268-273 | B828606 | 23.55 |

4. Humanization, cloning, expression, and purification of A10-268-42

The IMGT sorting method was used to divide A10-268-42 (42wt) into four framework regions and three CDR regions. AlphaFold2 was used to predict the 3D structure of 42wt, and a self-developed method was used to obtain the cardinality of this structure. Then, the 42wt sequence was compared with the IGHV3 sequences of 100 people using the IGBLAST application (https://www.ncbi.nlm.nih.gov/igblast/) to find the 14 non-human amino acids in the 4 Frameworks of A10-268-42. These 14 amino acids were divided into 13 groups (42hv1 to 42hv13), and 1 to 10 amino acids in each group were converted to human amino acids. Structures of these 13 VHHs with different degrees of humanized amino acid combinations were predicted by AlphaFold2. These 3D structures were compared with the 42wt structure, and the three combinations with the closest similarity (42hv10, 42hv11, and 42hv12) were selected. Together with 42wt itself, the above four VHHs were fused with Fc in which the 297th amino acid was mutated from N to A. Finally, the functions of these four proteins were compared through cellular cAMP experiments.

The C-termini of 42wt, 42hv10, 42hv11, and 42hv12 were respectively connected to the N-terminus of Fc to form fusion proteins (42wtFc, 42hv10Fc, 42hv11Fc, and 42hv12Fc), and gene synthesis, expression, and purification were entrusted to Biointron using the same methods as above. The protein electrophoresis diagram is shown in FIG. 4.

The sequences of 42hv10, 42hv11, 42hv12 are as follows:

QVQLVESGGGLVQPGGSLRLSCAAS**GRTFSGYV**MGWFRQAPGKGLEFVAA**LRWSSSNT**NYAASVKGRFTISRDYAK
DTVYLQMNSLRAEDTAVYYC**AAKRDRPYGSTWTPVAADYDT**WGQGTLVTVSS

42hv11 (SEQ ID NO: 62):

QVQLVESGGGLVQPGGSLRLSCAAS**GRTFSGYV**MGWFRQAPGKGLEFVAA**LRWSSSNT**NYADSVKGRFTISRDYAK
DTVYLQMNSLRAEDTAVYYC**AAKRDRPYGSTWTPVAADYDT**WGQGTLVTVSS

42hv12 (SEQ ID NO: 63):

QVQLVESGGGLVQPGGSLRLSCAAS**GRTFSGYV**MGWFRQAPGKGLEFVAA**LRWSSSNT**NYADSVKGRFTISRDNAK
DTVYLQMNSLRAEDTAVYYC**AAKRDRPYGSTWTPVAADYDT**WGQGTLVTVSS

Fc fragment in which the amino acid at position 297 is mutated from N to A (SEQ ID NO: 68)

EPKSADKTHTCPPCPAPELLGGPSVFLFPPKPKDTLMISRTPEVTCVVVDVSHEDPEVKFNWYVDGVEVH
NAKTKPREEQYASTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDKSRWQQGNVF
SCSVMHEALHNHYTQKSLSLSPGK

5. Verification of the cellular cAMP inhibition ability of humanized A10-268-42 (42hv10Fc)

On the day of the experiment, Neuroblastoma was separated from the culture flask with 0.25% Trypsin-EDTA, washed once with PBS, counted, and diluted to a cell solution with 5 million cells/mL with a test medium and used within 30 minutes.

5 $\mu$L of the cell solution and 5 $\mu$L of 42wtFc, 42hv10Fc, 42hv11Fc, and 42hv12Fc proteins and control antibody Erenumab (Biointron, A03EB) diluted 5-fold in series with a stimulation solution were added to the wells of the ProxiPlate-384 Plus test plate in sequence, with concentrations ranging from 0.0003 to 100 nM, and incubated at 37°C for 30 minutes.

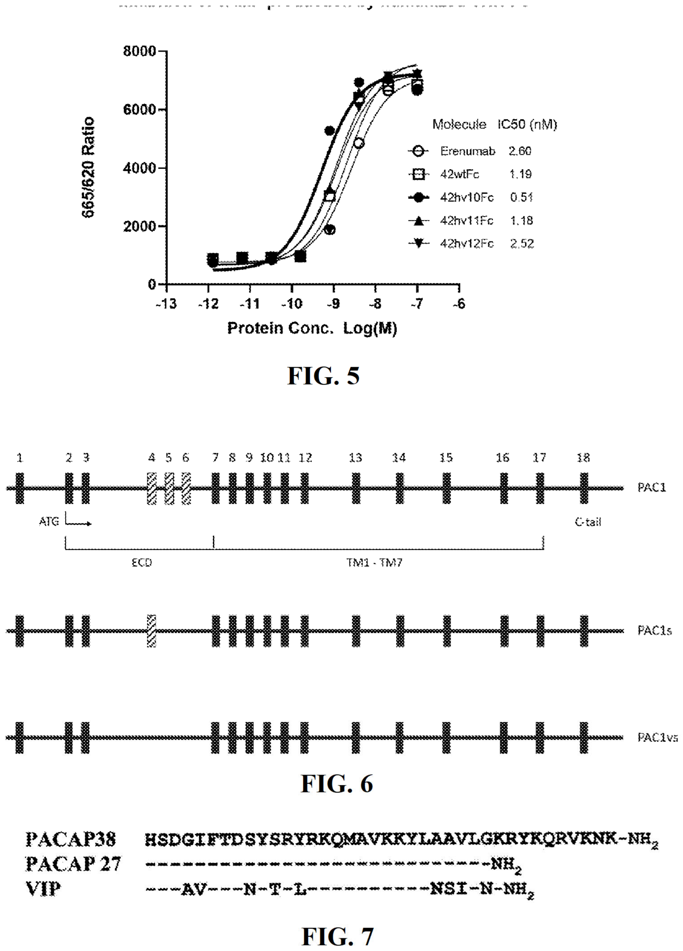

5 $\mu$L of CGRP diluted with a stimulation solution (final concentration is 5 nM) was added, the test plate was placed in a 37°C incubator for 15 minutes of incubation, and then 5 $\mu$L of cAMP-d2 and 5 $\mu$L of Anti-cAMP-Cryptate were added to terminate the reaction. The test plate was placed at room temperature for 60 minutes and then read using the 665/620 filter of a fluorescent microplate reader, the 665/620 ratio was calculated using the obtained values and then analyzed using the Nonlinear regression log (inhibitor) vs. response (three parameters) model in the GraphPad Prism 9.3.1 software, and the results are shown in FIG. 5. As can be seen from FIG. 5, the humanization of the three combinations of 42hv10, 42hv11, and 42hv12 were all successful, and the ability to inhibit cellular cAMP production was better than that of the control antibody Erenumab. Based on the above results, 42hv10 with $IC_{50}$ twice as good as the prototype was selected for further development.

**Example 3: Synthesis of the extracellular domain of pituitary adenylate cyclase 1 (PAC1 ECD)**

1. Gene synthesis, cloning, expression, and purification of PAC1 ECD and PACIs ECD-Fc fusion proteins

The full name of PAC1 is Pituitary Adenylate Cyelase 1, the Chinese name is pituitary adenylate cyclase 1, and the encoding gene is ADCYAP1R1. PAC1 is a membrane protein that belongs to the Class B G protein-coupled receptor (Class B GPCR), and it is widely expressed in animals, but is mainly distributed in endocrine organs and the nervous system. PAC1 and its ligand pituitary adenylate cyclase-activating polypeptide (PACAP, also known as PACAP38) constitute a signaling system that plays an important role in regulating growth, tissue repair, mood, metabolism, neuroprotection, etc.

ADCYAP1R1 has a total of 18 exons, 10 of which (exons 2, 3, 7-13, 18) are fixedly expressed, and the others are expressed through alternative splicing to form multiple isoforms, which are collectively referred to as isoforms. The amino terminus (N terminus) of the extracellular domain (ECD) of PAC1 is encoded by exons 2-6, the seven transmembrane regions (including the extracellular and intracellular parts) are encoded by exons 7-17, and the untranslated part of the carboxyl terminus (C terminus) is encoded by exon 18.

In humans, the N-terminal extracellular region (ECD) of PAC1 includes three isoforms: PAC1, PAC1s, and PAC1vs ( FIG. 6). If exons 2-6 are all expressed, it is PAC1; if exons 5 and 6 are cut out, it is PAC1s; if exons 4, 5, and 6 are cut out, it is PAC1vs.

The ligands of PAC1 include PACAP (PACAP38), PACAP27, and VIP (Vasoactive Intestinal Polypeptide) (FIG. 7). The affinities of PAC1 and PACAP are in the single nanomolar (nM) range, but the affinity for VIP is 100-1000-fold weaker. Although PAC1s lacks the 5th and 6th exons, it retains its affinity for its ligand PACAP, while its affinity for VIP is increased. The affinity of PAC1vs for ligands PACAP38 and PACAP27 are greatly reduced, but the affinity for VIP is retained.

The PACAP Trap is modified from the PAC1 ECD. PAC1 ECD is a soluble extracellular region of PAC1 that leaves the cell membrane, and it is found in the present invention that it also retains the ability to bind to PACAP in vitro. Moreover, PAC1 ECD without exons 5 and 6, namely PAC1s ECD, has a stronger ability to bind PACAP, so PAC1s ECD is used to construct PACAP Trap.

PAC1 ECD is from Genbank ID NM_001199635.2 nucleic acid sequence 506-907 (SEQ ID NO: 65), amino acid sequence 24-155 (SEQ ID NO: 64).

The nucleic acid sequence is:

cattctgactgcatcttcaagaaggagcaagccatgtgcctggagaagatccagagggccaatgagctgatgggcttcaatgattcctctccaggctgtcctggatg tgggacaacatcacgtgttggaagcccgcccatgtgggtgagatggtcctggtcagctgccctgagctcttccgaatcttcaacccagaccaagtctgggagaccga aaccattggagagtctgattttggtgacagtaactccttagatctctcagacatggagtggtgagccggaactgcacggaggatggctggtcggaacccttccctcat tactttgatgcctgtgggtttgatgaatatgaatctgagactgggggaccaggattattactacctgtcagtgaaggcc

The amino acid sequence is:

MHSDCIFKKEQAMCLEKIQRANELMGFNDSSPGCPGMWDNITCWKPAHVGEMVLVSCPELFRIFNPDQ
VWETETIGESDFGDSNSLDLSDMGVVSRNCTEDGWSEPFPHYFDACGFDEYESETGDQDYYYLSVKA

The nucleic acid sequence used for cloning PAC1s ECD is from Genbank ID NM 001199637.2 506-844 (SEQ ID NO: 67), which is:

Atgcattctgactgcatcttcaagaaggagcaagccatgtgcctggagaagatccagagggccaatgagctgatgggcttcaatgattcctctccaggctgtcctgg gatgtgggacaacatcacgtgttggaagcccgcccatgtgggtgagatggtcctggtcagctgccctgagctcttccgaatcttcaacccagaccaagcagacatgg gagtggtgagccggaactgcacggaggatggctggtcggaacccttccctcattactttgatgcctgtgggtttgatgaatatgaatctgagactgggggaccaggatta ttactacctgtcagtgaaggccct

The amino acid sequence of PAC1s ECD (or with addition: from UniProt ID P41586-3 21-124) (SEQ ID NO: 66), which is:

MHSDCIFKKEQAMCLEKIQRANELMGFNDSSPGCPGMWDNITCWKPAHVGEMVLVSCPELFRIFNPDQ
DMGVVSRNCTEDGWSEPFPHYFDACGFDEYESETGDQDYYYLSVKA

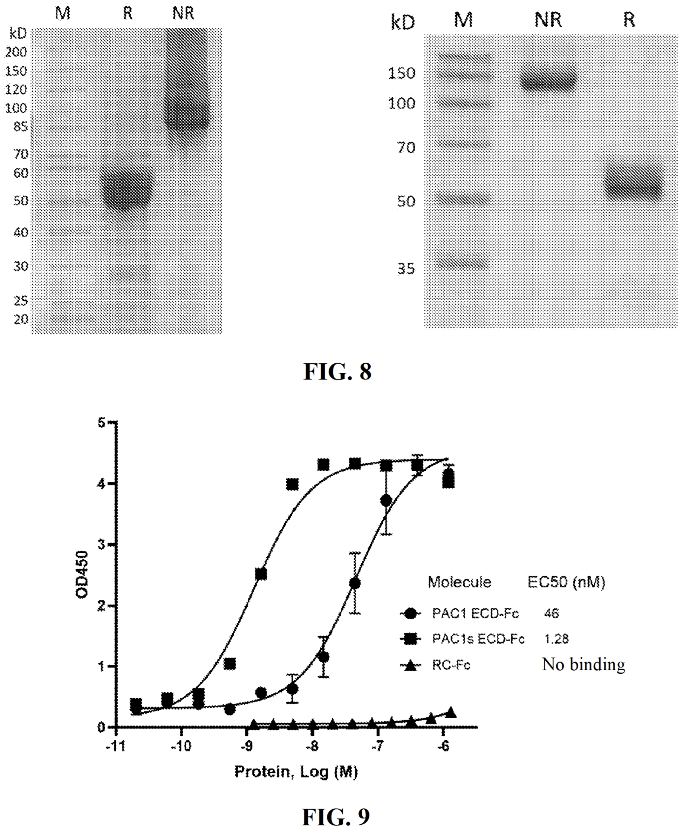

The C-terminus of PAC1 ECD and PAC1s ECD and the N-terminus of Fc were fused and expressed as PAC1 ECD-Fc and PACs1 ECD-Fc. Gene synthesis and expression were entrusted to Biointron, and the same methods as above were used. SDS-PAGE electrophoresis identification is shown in FIG. 8.

2. ELISA evaluation of the ability of PAC1 ECD-Fc and PAC1s ECD-Fc to bind to PACAP

After the streptavidin-coated 96-well ELISA plate (ThermoFisher Scientific™ 15126) was washed three times with PBST (0.1% Tween 20, pH=7.4), 1000 ng of Biotin-PACAP was added to each well, and incubated at room temperature for 2 hours. PBST was removed, and 100 $\mu$L of PAC1 ECD-Fe, PAC1s ECD-Fc, or negative control RC-Fc fusion protein serially diluted with PBST was added to each well, and incubated at 37°C for 1 hour. The ELISA plate incubated with the samples was washed five times with PBST, then 100 $\mu$L of the corresponding secondary antibody Anti-human IgG-HRP (anti-human immunoglobulin G-horseradish peroxidase, Hangzhou HuaBio, HR1214) diluted 1:5000 with a blocking solution was added, and incubated at 37°C for 1 hour.

The ELISA plate incubated with the secondary antibody was washed 5 times with PBST, 100 $\mu$L of TMB (Trimethylolpropane) single-component colorimetric solution (Solarbio, 20190402) was added to each well, incubated at 37°C for 7 min, 100 $\mu$L of 1M HCL (Kelong Chemicals) was added to each well to terminate the reaction, and finally, the OD450 reading was taken with an ELISA reader. The results were analyzed using the Nonlinear regression log (agonist) vs. response (three parameters) model. The results are shown in FIG. 9. As can be seen in the figure, the ability of PAC1s ECD-Fc to bind PACAP is 40 times that of PAC1 ECD-Fc, while the extracellular region RC-Fc of the CGRP receptor has no binding ability.

**Example 4: Construction of 42hv10FcNA-PAC1s ECD (BY003) bifunctional fusion protein**

1. Cloning, expression, and purification of BY003

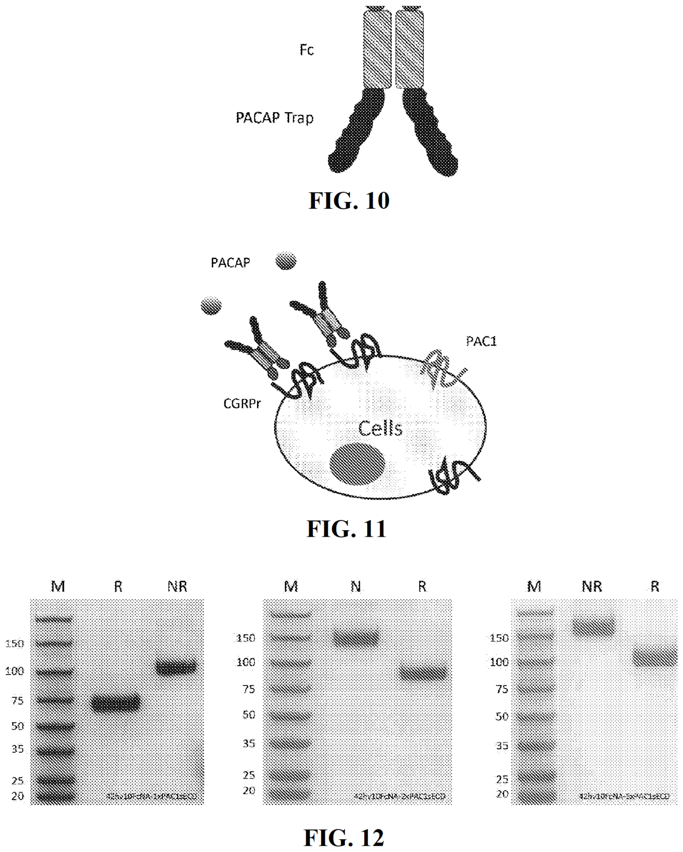

BY003 comprises a fusion of three molecules: 42hv10, FcNA (the amino acid at position 297 of Fc is mutated from N to A), and PAC1s ECD. The C-terminus of 42hv10 was connected to the N-terminus of FcNA, and the C-terminus of FcNA was connected to PAC1s ECD via the linker GGGGS (SEQ ID NO: 79). There may be one, two, or three PAC1s ECDs. In the case of multiple PAC1s ECDs, the PAC1s ECDs were connected to each other by GGGGS. The BY003 structure consisting of two PAC1s ECDs is shown in FIG. 10, and the working principle of BY003 is shown in FIG. 11.

Gene synthesis was entrusted to Biointron, and the same method as above was used. SDS-PAGE electrophoresis identification is shown in FIG. 12.

2. Cell function verification of anti-42hv10 in BY003 (inhibition of cAMP production in CHO-CGRPrC3 cells induced by CGPR)

In the present example, the verification indicator of cell function is the ability of BY003 to inhibit the production of cAMP.

2.1 Establishment and culture of cell lines expressing CGRP receptors

The CHO cell pool stably expressing human Ramp1 ECD and CLR ECD (referred to as CHO-hCGRPr or CHO-A10 cells) was obtained from Taizhou Biointron Biotechnology Co., Ltd. with the catalog number CHOK1-C21738001. The culture medium is DMEM/F-12+10% FBS+1x penicillin-streptomycin mixture (Solarbio, P1400) + 10 $\mu$g/mL puromycin (Solarbio, P8230) + 50 ug/ml hygromycin B (Solarbio, H8080), which is referred to as CHO-hCGRPr culture medium.
The CHO-hCGRPr cell pool was diluted to 5 cells/mL with the CHO-hCGRPr culture medium, and then 100 $\mu$L of the diluted cell solution was transferred to the wells of two 96-well cell culture plates (Thermo Scientific, 167008) and cultured in a CO2 incubator until individual cell colonies could be differentiated. Six colonies were transferred from the 96-well plate to a 6-well cell culture plate (Corning, 3516). After growing to 90% confluence, the cells were transferred to T-75 (Corning, 430541U) cell culture flasks. Once the cells reached full confluence, they could be used for functional testing and cryopreservation in liquid nitrogen.
The cellular cAMP concentration was tested using the cAMP-GS Dynamic Kit (Cisbio, 62AM4PEB) from Cisbio. Unless otherwise specified, the operation method was carried out according to the instructions of the kit. The test culture medium was DMEM/F1-12+10% FBS+1 mM IBMX), and the test ELISA plate was a white 384-well plate.

2.2 Acquisition of the CGRP standard curves of monoclonal stable cell lines

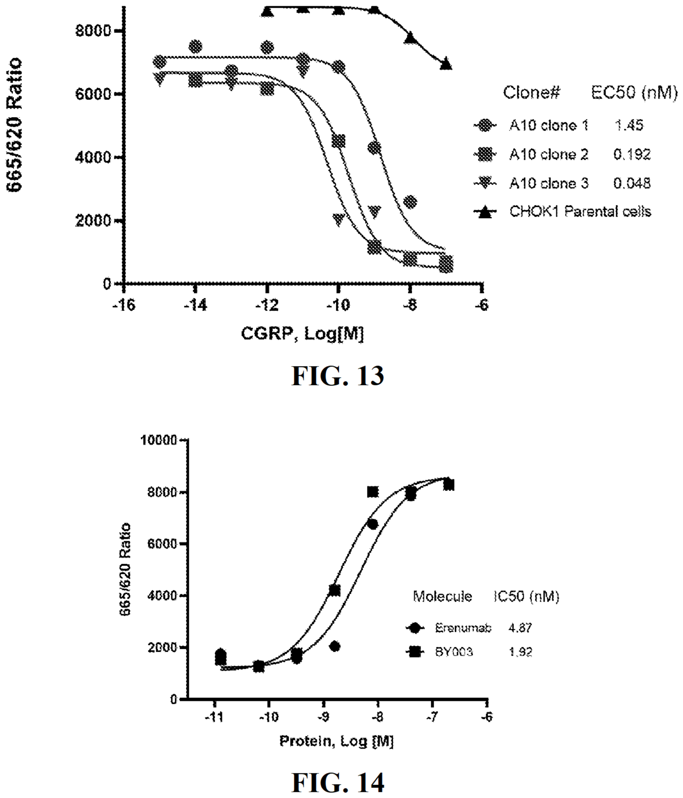

From the above six single-cell clones, clones 1, 2, and 3 were selected to evaluate their ability to produce cAMP, and CHOK1 parent cell line (ECACC, 85051005) not transfected with CGRP receptor was added as a control. On the day of the experiment, the cells were separated from the culture flask with 0.25% Trypsin-EDTA (Gibco, 25200-056), washed once with PBS, counted, and diluted to a cell solution with 5 million cells/mL with a test medium and used within 30 minutes.
5 $\mu$L of the cell solution and 5 $\mu$L of CGRP (Nanjing Yuan-Peptide Biotechnology Co., Ltd., A03-137, batch number: yuanpeptide-997792) diluted with a stimulation buffer (Stimulation Buffer + 500 $\mu$M IBMX) were added to the wells of the ProxiPlate-384 Plus test plate in sequence. The final concentration range of CGRP was 0 nM or 0.000001-100 nM, and diluted in 10-fold series.
After adding CGRP, the test plate was incubated in a 37°C incubator for 15 minutes, and then 5 $\mu$L cAMP-d2 and 5 $\mu$L Anti-cAMP-Cryptate were added to terminate the reaction. The test plate was left at room temperature for 60 minutes and then read using a fluorescence microplate reader (BMG LabTech, Model: PHERAstar FSX) with a 665/620 filter. The obtained values were calculated using Microsoft Excel to obtain the 665/620 ratio, and finally analyzed using the lNonlinear regression log (agonist) vs. response (three parameters) model in the GraphPad Prism 9.3.1 software. The results are shown in FIG. 13. Based on the results, the cell line 3 was selected for subsequent experiments and was named CHO-CGRPrC3.

2.3 Evaluation of the ability of BY003 to inhibit CGPR-induced cAMP production in CHO-CGRPrC3 cells

On the day of the experiment, the CHO-hCGRPrC3 cells were separated from the culture flask with 0.25% Trypsin-EDTA, washed once with PBS, counted, and diluted to a cell solution with 5 million cells/mL with a test medium and used within 30 minutes.
5 $\mu$L of the cell solution and 5 $\mu$L of BY003 and control antibody Erenumab (Biointron, A03EB) diluted 5-fold in series with a stimulation solution were added to the wells of the ProxiPlate-384 Plus test plate in sequence, with concentrations ranging from 0.0003 to 100 nM, and incubated at 37°C for 30 minutes.
5 $\mu$L of CGRP diluted with a stimulation solution (final concentration is 1.4 nM) was added, the test plate was placed in a 37°C incubator for 15 minutes of incubation, and then 5 $\mu$L of cAMP-d2 and 5 $\mu$L of Anti-cAMP-Cryptate were added to terminate the reaction. The test plate was placed at room temperature for 60 minutes and then read using a 665/620 filter of a fluorescent microplate reader, the 665/620 ratio was calculated using the obtained values and then analyzed using the Nonlinear regression log (inhibitor) vs. response (three parameters) model in the GraphPad Prism 9.3.1 software, and the results are shown in FIG. 14. As can be seen in the figure, the ability of BY003 to inhibit the production of cAMP in CHO-hCGRPrC3 cells is 2.5 times stronger than that of Erenumab.

3. Cell function verification of anti-42hv10 in BY003 (inhibition of cAMP production in CHO-hPAC1 cells induced by CGPR)

3.1 Establishment and culture of stable cell lines expressing human PAC1

The cell pools stably expressing human PAC1 ECD and PAC1s ECD CHO (referred to as CHO-A05 and CHO-A05s cells) were obtained from Taizhou Biointron Biotechnology Co., Ltd. with the catalog numbers CHOK1-A05 and CHOK1-A05s. The culture medium is DMEM/F-12+10% FBS+1x penicillin-streptomycin mixture (Solarbio, P1400) + 10 μg/mL puromycin (Solarbio, P8230), which is referred to as CHO-PAC1 culture medium.

The CHO-A05 and CHO-A05s cell pool was diluted to 5 cells/mL with the CHO-PAC1 culture medium, and then 100 μL of the diluted cell solution was transferred to the wells of two 96-well cell culture plates (Thermo Scientific, 167008) and cultured in a $CO_2$ incubator until individual cell colonies could be differentiated. Six colonies were transferred from the 96-well plate to a 6-well cell culture plate (Corning, 3516). After growing to 90% confluence, the cells were transferred to T-75 (Corning, 430541U) cell culture flasks. Once the cells reached full confluence, they could be used for testing and cryopreservation in liquid nitrogen.

3.2 Acquisition of the cAMP standard curves of the monoclonal CHO-A05 cell line

On the day of the experiment, six monoclonal CHO-A05 cells and CHOK1 parent cell line (ECACC, 85051005) not transfected with PAC1 were isolated from the culture flask with 0.25% Trypsin-EDTA, washed once with PBS, counted, and the cells were diluted to a cell solution with 5 million cells/mL with a test medium and used within 30 minutes.

5 μL of the cell solution and 5 μL of PACAP (Nanjing Yuan-Peptide Biotechnology Co., Ltd., A05-138, batch number: Yuanpeptide-194972) diluted with a stimulation buffer (Stimulation Buffer + IBMX) were added to the wells of the ProxiPlate-384 Plus test plate in sequence. The final concentration of PACAP ranged from 0.0001 to 100 nM.

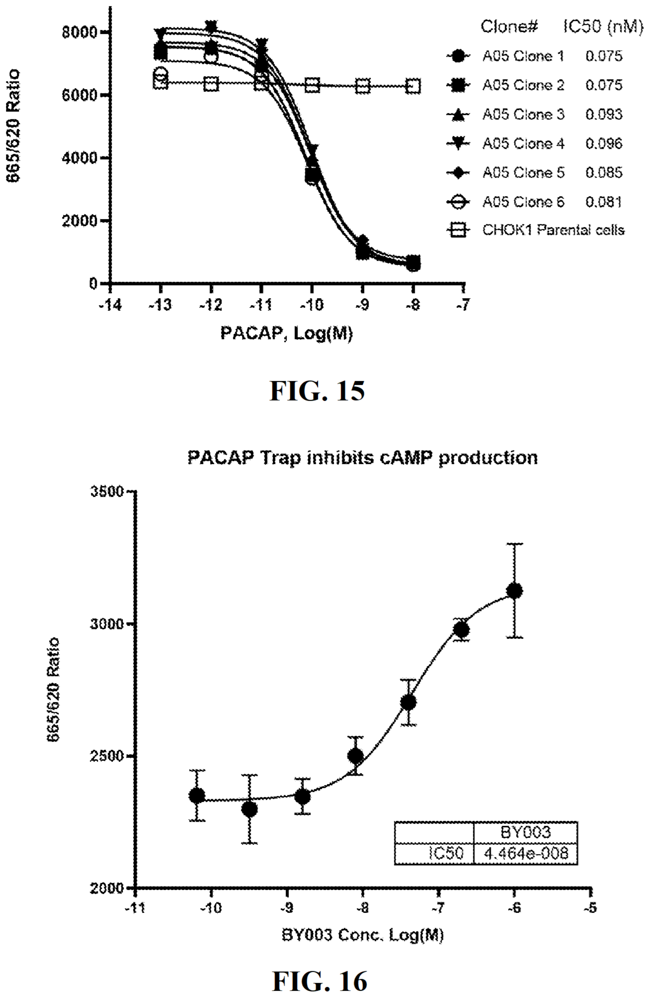

After adding PACAP, the test plate was incubated in a 37°C incubator for 15 minutes, and then 5 μL cAMP-d2 and 5 μL Anti-cAMP-Cryptate were added to terminate the reaction. The test plate was placed at room temperature for 60 minutes and then read using a 665/620 filter on a fluorescent microplate reader (BMG LabTech, Model: PHERAstar FSX). The obtained values were calculated using Microsoft Excel to obtain the 665/620 ratio, and finally analyzed using the Nonlinear regression log (agonist) vs. response (three parameters) model in the GraphPad Prism 9.3.1 software. The results are shown in FIG. 15. Based on the results, the cell line 4 was selected for subsequent experiments and was named CHO-hPAC1C4.

3.3 Evaluation of the ability of anti-BY003 to inhibit cAMP production in CHO-hPAC1C4 cells

The cellular cAMP concentration was tested using the cAMP Kit from Cisbio. Unless otherwise specified, the operation method was carried out according to the instructions of the kit. The test culture medium was DMEM/F1-12+10% FBS+1 mM IBMX), and the test ELISA plate was a white 384-well plate.

Since BY003 is a bifunctional protein that acts on both the CGRP receptor and PACAP, CHO-hCGRPrC3 and CHO-hPAC1C4 cells were added to the experimental test plate. One day before the experiment, the above two types of cells were separated from the culture flask with 0.25% Trypsin-EDTA, washed once with PBS, counted, and diluted to a cell solution with 4 million cells/mL with a test culture medium. After mixing the two cell solutions, 10 μL of the mixture was transferred to a 384-well cell culture plate (Corning, 3765) and placed in a 37°C $CO_2$ incubator overnight.

On the day of the experiment, the culture medium was removed, and 5 μL of BY003 single-domain antibody serially diluted with a stimulation solution (final concentration was 0 nM or 0.1-1000 nM) was added to the wells of the test plate and incubated in a 37°C incubator for 30 minutes.

5 μL of PACAP diluted with a stimulation solution (final concentration is 0.7 nM) was added, the test plate was placed in a 37°C incubator for 15 minutes of incubation after PACAP was added, and then 5 μL cAMP-d2 and 5 μL Anti-cAMP-Cryptate were added to terminate the reaction. The test plate was left at room temperature for 60 minutes and then read using a fluorescence microplate reader (BMG LabTech, Model: PHERAstar FSX) with a 665/620 filter. The obtained values were calculated using Microsoft Excel to obtain the 665/620 ratio, and finally analyzed using the Nonlinear regression log (agonist) vs. response (three parameters) model in the GraphPad Prism 9.3.1 software. The results are shown in FIG. 16.

In response to the unmet clinical needs in the current field of migraine treatment and prevention, the present invention provides a fusion protein that simultaneously blocks the CGRP pathway and the PACAP pathway. By using the method of fusing a single-domain VHH against the CGRP receptor with natural PAC1 soluble EDC, the CGRP and PACAP signaling pathways, both of which are closely related to migraine, are blocked simultaneously. The present invention has successfully constructed BY003 molecules and proved that BY003 can block the production of cAMP induced by CGRP and PACAP at the cellular level.

**Claims**

1.  A single-domain antibody for blocking the calcitonin gene-related peptide (CGRP) pathway, wherein the single-domain antibody comprises a CDR1 as shown in the amino acid sequence of SEQ ID NO: 2, 7, 12, 17, 22, 27, 32, 37, 42, 47, 52, or 57 or any variant thereof, a CDR2 as shown in the amino acid sequence of SEQ ID NO: 3, 8, 13, 18, 23, 28, 33, 38, 43, 48, 53, or 58 or any variant thereof, and a CDR3 as shown in the amino acid sequence of SEQ ID NO: 4, 9, 14, 19, 24, 29, 34, 39, 44, 49, 54, or 59 or any variant thereof;
    preferably, the single-domain antibody comprises CDR1 as shown in the amino acid sequence of SEQ ID NO: 12 or any variant thereof, CDR2 as shown in the amino acid sequence of SEQ ID NO: 13 or any variant thereof, and CDR3 as shown in the amino acid sequence of SEQ ID NO: 14 or any variant thereof.

2.  The single-domain antibody according to claim 1, wherein the single-domain antibody comprises a variable region as shown in the amino acid sequence of SEQ ID NO: 1, 6, 11, 16, 21, 26, 31, 36, 41, 46, 51, 56, 61, 62 or 63 or any variant thereof;

    preferably, the single-domain antibody comprises a variable region as shown in the amino acid sequence of SEQ ID NO: 61, 62, or 63 or any variant thereof;
    more preferably, the single-domain antibody comprises a variable region as shown in the amino acid sequence of SEQ ID NO: 61 or any variant thereof;
    preferably, the amino acids in the single-domain antibody have conservative substitutions, and the number of conservatively substituted amino acids does not exceed 1, 2, 3, 4, 5, 6, 7, 8, 9, or 10.

3.  A fusion protein, which simultaneously blocks the calcitonin gene-related peptide (CGRP) pathway and the pituitary adenylate cyclase-activating peptide (PACAP) pathway, wherein the fusion protein comprises at least one single-domain antibody, at least one Fc fragment, and at least one extracellular domain fragment of pituitary adenylate cyclase 1 (PAC1 ECD).

4.  The fusion protein according to claim 3, wherein the fusion protein comprises one peptide chain, the structure of the peptide chain is that one PAC1 ECD is connected to the C-terminus of an Fc fragment via a linker, and the N-terminus of the Fc fragment is fused to the C-terminus of a single-domain antibody;

    optionally, the fusion protein comprises two peptide chains, the structure of the peptide chains is that one PAC1 ECD is connected to the C-terminus of an Fc fragment via a linker, and the N-terminus of the Fc fragment is fused to the C-terminus of a single-domain antibody, and two PAC1 ECDs of the two peptide chains are connected via a linker;
    optionally, the fusion protein comprises three peptide chains, the structure of which is that one PAC1 ECD is connected to the C-terminus of an Fc fragment via a linker, and the N-terminus of the Fc fragment is fused to the C-terminus of a single-domain antibody, and three PAC1 ECDs of the three peptide chains are connected via a linker.

5.  The fusion protein according to claim 3 or 4, wherein the single-domain antibody is a single-domain antibody that binds to a CGRP receptor;

    preferably, the single-domain antibody comprises a CDR1 as shown in the amino acid sequence of SEQ ID NO: 2, 7, 12, 17, 22, 27, 32, 37, 42, 47, 52, or 57 or any variant thereof, a CDR2 as shown in the amino acid sequence of SEQ ID NO: 3, 8, 13, 18, 23, 28, 33, 38, 43, 48, 53, or 58 or any variant thereof, and a CDR3 as shown in the amino acid sequence of SEQ ID NO: 4, 9, 14, 19, 24, 29, 34, 39, 44, 49, 54, or 59 or any variant thereof;
    more preferably, the single-domain antibody comprises CDR1 as shown in the amino acid sequence of SEQ ID NO: 12 or any variant thereof, CDR2 as shown in the amino acid sequence of SEQ ID NO: 13 or any variant thereof, and CDR3 as shown in the amino acid sequence of SEQ ID NO: 14 or any variant thereof;
    preferably, the amino acids in one or more or all three of the CDRs have conservative substitutions, and the number of conservatively substituted amino acids does not exceed 1, 2, 3, 4, 5, 6, 7, 8, 9, or 10.

6.  The fusion protein according to any one of claims 3 to 5, wherein the single-domain antibody comprises a variable region as shown in the amino acid sequence of SEQ ID NO: 1, 6, 11, 16, 21, 26, 31, 36, 41, 46, 51, 56, 61, 62 or 63 or any variant thereof;

    preferably, the single-domain antibody comprises a variable region as shown in the amino acid sequence of SEQ ID NO: 61, 62, or 63 or any variant thereof;
more preferably, the single-domain antibody comprises a variable region as shown in the amino acid sequence of SEQ ID NO: 61 or any variant thereof;
preferably, the amino acids in the single-domain antibody have conservative substitutions, and the number of conservatively substituted amino acids does not exceed 1, 2, 3, 4, 5, 6, 7, 8, 9, or 10.

7. The fusion protein according to any one of claims 3 to 6, wherein the PAC1 ECD is selected from the ECD of the PAC isoforms: PAC1, PAC1s, or PAC1vs;

preferably, the PAC1 ECD is PAC1s ECD;
preferably, the PAC1 ECD comprises the amino acid sequence of SEQ ID NO: 64 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto;
preferably, the PAC1 ECD comprises the amino acid sequence of SEQ ID NO: 66 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto.

8. The fusion protein according to any one of claims 3 to 7, wherein the Fc fragment comprises a hinge region and constant region 2 (CH2) and constant region 3 (CH3) domains derived from a human immunoglobulin;

preferably, the Fc fragment is selected from human IgG1, IgG2, IgG3, and IgG4; more preferably, the Fc fragment is selected to be human IgG1;
preferably, the Fc fragment has an amino acid substitution; more preferably, the amino acid substitution includes replacement of asparagine N at position 297 on the Fc fragment by alanine A, wherein each amino acid site on the Fc fragment is numbered using the EU antibody numbering system;
more preferably, the Fc fragment comprises the amino acid sequence of SEQ ID NO: 68 and an amino acid sequence having 80% or higher identity thereto, preferably an amino acid sequence having 85%, 90%, 95%, 96%, 97%, 98%, 99%, or higher identity thereto, and more preferably an amino acid sequence having 98% or 99% or higher identity thereto.

9. The fusion protein according to any one of claims 3 to 8, wherein the linker is a flexible polypeptide linker;

preferably, the flexible polypeptide linker is selected from GGGGS (SEQ ID NO: 79), GGGGSGGGGS (SEQ ID NO: 80), SGGGGSGGGG (SEQ ID NO: 81), GGGGSGGGGSSGGGGS (SEQ ID NO: 82), GGGGSGGGGSGGGGS (SEQ ID NO: 83), GGGGSGGGGSGGGG, (SEQ ID NO: 84), GGGGSGGGGSGGGGSGGGGS (SEQ ID NO: 85), GGGGSGGGGSGGGGSGGGGS (SEQ ID NO: 86), GSGSGSGS (SEQ ID NO: 87), GGSGSGSG (SEQ ID NO: 88), GGSGSG (SEQ ID NO: 89), and GGSG (SEQ ID NO: 90);
more preferably, the flexible polypeptide linker is GGGGS (SEQ ID NO: 79).

10. A nucleic acid encoding the single-domain antibody according to claim 1 or 2 and the fusion protein according to any one of claims 3 to 9.

11. A vector comprising the nucleic acid according to claim 10.

12. A cell comprising the nucleic acid according to claim 10 or the vector according to claim 11.

13. A pharmaceutical composition or kit comprising the single-domain antibody according to claim 1 or 2 and/or the fusion protein according to any one of claims 3 to 9, as well as a pharmaceutically acceptable vector, diluent, or excipient.

14. Use of the single-domain antibody according to claim 1 or 2, the fusion protein according to any one of claims 3 to 9, the nucleic acid according to claim 10, the vector according to claim 11, and/or the cell according to claim 12 and/or the pharmaceutical composition or kit according to claim 13 in the preparation of a drug for treating a disease associated with activation of the CGRP and PACAP pathways;
preferably, the disease is migraine.

**FIG. 1**

**RC-Fc Binding to CGRP**

|  | CGRP | PACAP |
|---|---|---|
| One site -- Specific binding |  |  |
| Best-fit values |  |  |
| Bmax | 5.198 | 0.06873 |
| Kd | 16.12 | 0.5274 |
| 95% CI (profile likelihood) |  |  |
| Bmax | 4.711 to 5.741 | 0.06195 to 0.07589 |
| Kd | 11.83 to 21.95 | 0.08463 to 1.213 |

**FIG. 2**

**FIG. 3**

**FIG. 4**

**FIG. 5**

**FIG. 6**

PACAP38   HSDGIFTDSYSRYRKQMAVKKYLAAVLGKRYKQRVKNK~NH₂
PACAP 27  ----------------------------------------~NH₂
VIP       ---AV---N-T-L------------NSI-N-NH₂

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

## INTERNATIONAL SEARCH REPORT

| International application No. |
| --- |
| **PCT/CN2023/143105** |

| A. | CLASSIFICATION OF SUBJECT MATTER |
| --- | --- |

C07K 16/28(2006.01)i;  C07K 16/46(2006.01)i;  A61K39/00(2006.01)i

According to International Patent Classification (IPC) or to both national classification and IPC

| B. | FIELDS SEARCHED |
| --- | --- |

Minimum documentation searched (classification system followed by classification symbols)

IPC: C07K, A61K

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, ISI Web of Knowledge, PubMed, CNKI, GenBank, 中国专利序列数据库, Chinese Patent Sequence Retrieval System: SEQ ID NOs: 1-90, 降钙素基因相关肽, CGRP, 垂体腺苷酸环化酶激活肽, PACAP, 胞外域, ECD, 单域抗体, single domain antibody, 纳米抗体, nanobody, 重链可变区, VH, 轻链可变区, VL, 头痛, headache, 偏头痛, migraine

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2017306033 A1 (AMGEN INC.) 26 October 2017 (2017-10-26) claims 1-102 | 3-5, 7-14 |
| X | US 2022363770 A1 (AMGEN INC.) 17 November 2022 (2022-11-17) claims 1-70 | 3-5, 7-14 |
| A | CN 108137677 A (ALDER BIOPHARMACEUTICALS INC.) 08 June 2018 (2018-06-08) claims 1-89 | 1-14 |
| A | CN 110300595 A (THE REGENTS OF THE UNIVERSITY OF CALIFORNIA et al.) 01 October 2019 (2019-10-01) claims 1-77 | 1-14 |
| A | CN 111566126 A (AMGEN INC.) 21 August 2020 (2020-08-21) claims 1-56 | 1-14 |
| A | PDB: 6LPB_R. "Chain R, Pituitary adenylate cyclase-activating polypeptide type I receptor" *GenBank*, 01 December 2020 (2020-12-01), sections FEATURES, and ORIGIN | 1-14 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **09 April 2024** | **15 April 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/143105**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☐  forming part of the international application as filed.

    b.  ☑  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

| International application No. |
| --- |
| **PCT/CN2023/143105** |

| Patent document cited in search report | | | Publication date (day/month/year) | Patent family member(s) | | | Publication date (day/month/year) |
| --- | --- | --- | --- | --- | --- | --- | --- |
| US | 2017306033 | A1 | 26 October 2017 | AR | 101875 | A1 | 18 January 2017 |
| | | | | AU | 2015318008 | A1 | 23 March 2017 |
| | | | | AU | 2015318008 | B2 | 20 May 2021 |
| | | | | CA | 2960756 | A1 | 24 March 2016 |
| | | | | CA | 2960756 | C | 01 August 2023 |
| | | | | US | 10934362 | B2 | 02 March 2021 |
| | | | | JP | 2017553694 | A | 16 November 2017 |
| | | | | JP | 6956631 | B2 | 02 November 2021 |
| | | | | JP | 2020074790 | A | 21 May 2020 |
| | | | | EP | 3194435 | A1 | 26 July 2017 |
| | | | | US | 2021047422 | A1 | 18 February 2021 |
| | | | | US | 11919964 | B2 | 05 March 2024 |
| | | | | TW | 201620938 | A | 16 June 2016 |
| | | | | MX | 2017003247 | A | 30 November 2017 |
| | | | | UY | 36302 | A | 29 April 2016 |
| | | | | WO | 2016044224 | A1 | 24 March 2016 |
| US | 2022363770 | A1 | 17 November 2022 | WO | 2020264384 | A1 | 30 December 2020 |
| | | | | JP | 2022538232 | A | 01 September 2022 |
| | | | | CA | 3143524 | A1 | 30 December 2020 |
| | | | | MX | 2021015791 | A | 01 April 2022 |
| | | | | AU | 2020304671 | A1 | 20 January 2022 |
| | | | | EP | 3990493 | A1 | 04 May 2022 |
| CN | 108137677 | A | 08 June 2018 | JP | 2018516071 | A | 21 June 2018 |
| | | | | JP | 6924148 | B2 | 25 August 2021 |
| | | | | US | 2021324068 | A1 | 21 October 2021 |
| | | | | CA | 2983041 | A1 | 20 October 2016 |
| | | | | IL | 254954 | A0 | 31 December 2017 |
| | | | | IL | 254954 | B | 31 October 2021 |
| | | | | WO | 2016168757 | A1 | 20 October 2016 |
| | | | | AU | 2016249140 | A1 | 09 November 2017 |
| | | | | AU | 2016249140 | B2 | 24 March 2022 |
| | | | | AU | 2016249140 | A8 | 21 July 2022 |
| | | | | US | 2019270807 | A1 | 05 September 2019 |
| | | | | US | 10981984 | B2 | 20 April 2021 |
| | | | | JP | 2023052620 | A | 11 April 2023 |
| | | | | US | 2016362488 | A1 | 15 December 2016 |
| | | | | US | 2020079847 | A1 | 12 March 2020 |
| | | | | US | 2021221884 | A9 | 22 July 2021 |
| | | | | US | 11254741 | B2 | 22 February 2022 |
| | | | | HK | 1256381 | A1 | 20 September 2019 |
| | | | | JP | 2018516361 | A | 21 June 2018 |
| | | | | JP | 6752222 | B2 | 09 September 2020 |
| | | | | US | 2021380676 | A1 | 09 December 2021 |
| | | | | US | 2016376363 | A1 | 29 December 2016 |
| | | | | CA | 2982861 | A1 | 20 October 2016 |
| | | | | US | 2022289836 | A1 | 15 September 2022 |
| | | | | WO | 2016168768 | A2 | 20 October 2016 |
| | | | | WO | 2016168768 | A3 | 01 December 2016 |
| | | | | US | 2016361441 | A1 | 15 December 2016 |
| | | | | US | 10844116 | B2 | 24 November 2020 |

Form PCT/ISA/210 (patent family annex) (July 2022)

## INTERNATIONAL SEARCH REPORT
### Information on patent family members

| International application No. |
| --- |
| **PCT/CN2023/143105** |

| Patent document cited in search report | | | Publication date (day/month/year) | Patent family member(s) | | | Publication date (day/month/year) |
| --- | --- | --- | --- | --- | --- | --- | --- |
| | | | | KR | 20180002685 | A | 08 January 2018 |
| | | | | JP | 2018512861 | A | 24 May 2018 |
| | | | | JP | 6917899 | B2 | 11 August 2021 |
| | | | | AU | 2016249406 | A1 | 09 November 2017 |
| | | | | AU | 2016249406 | B2 | 10 June 2021 |
| | | | | IL | 254955 | A0 | 31 December 2017 |
| | | | | IL | 254955 | B | 30 September 2021 |
| | | | | EP | 3283169 | A1 | 21 February 2018 |
| | | | | EP | 3283169 | A4 | 20 March 2019 |
| | | | | US | 2020172612 | A1 | 04 June 2020 |
| | | | | US | 10981985 | B2 | 20 April 2021 |
| | | | | EP | 3283516 | A2 | 21 February 2018 |
| | | | | EP | 3283516 | A4 | 06 March 2019 |
| | | | | AU | 2016249408 | A1 | 09 November 2017 |
| | | | | TW | 201702266 | A | 16 January 2017 |
| | | | | TWI | 721976 | B | 21 March 2021 |
| | | | | IL | 254945 | A0 | 31 December 2017 |
| | | | | IL | 254945 | B | 30 September 2021 |
| | | | | EP | 3283517 | A2 | 21 February 2018 |
| | | | | EP | 3283517 | A4 | 06 March 2019 |
| | | | | WO | 2016168762 | A2 | 20 October 2016 |
| | | | | WO | 2016168762 | A3 | 22 December 2016 |
| | | | | JP | 2018518659 | A | 12 July 2018 |
| | | | | JP | 6752223 | B2 | 09 September 2020 |
| | | | | US | 2021277106 | A1 | 09 September 2021 |
| | | | | US | 2016304604 | A1 | 20 October 2016 |
| | | | | US | 10899834 | B2 | 26 January 2021 |
| | | | | JP | 2021177772 | A | 18 November 2021 |
| | | | | JP | 7219308 | B2 | 07 February 2023 |
| | | | | CA | 2983030 | A1 | 20 October 2016 |
| | | | | EP | 3283168 | A1 | 21 February 2018 |
| | | | | EP | 3283168 | A4 | 06 March 2019 |
| | | | | WO | 2016168760 | A1 | 20 October 2016 |
| | | | | MX | 2017013113 | A | 06 July 2018 |
| | | | | KR | 20180002684 | A | 08 January 2018 |
| | | | | CA | 2982856 | A1 | 20 October 2016 |
| | | | | TW | 201643198 | A | 16 December 2016 |
| | | | | TWI | 721975 | B | 21 March 2021 |
| CN | 110300595 | A | 01 October 2019 | EP | 3565573 | A2 | 13 November 2019 |
| | | | | EP | 3565573 | A4 | 12 August 2020 |
| | | | | EP | 3565573 | B1 | 23 August 2023 |
| | | | | US | 2022251162 | A1 | 11 August 2022 |
| | | | | WO | 2018129200 | A2 | 12 July 2018 |
| | | | | WO | 2018129200 | A3 | 09 August 2018 |
| | | | | JP | 2020503367 | A | 30 January 2020 |
| | | | | JP | 7183164 | B2 | 05 December 2022 |
| | | | | MX | 2019008056 | A | 21 November 2019 |
| | | | | AU | 2018205458 | A1 | 11 July 2019 |
| | | | | US | 2019322718 | A1 | 24 October 2019 |
| | | | | US | 11186622 | B2 | 30 November 2021 |

Form PCT/ISA/210 (patent family annex) (July 2022)

**INTERNATIONAL SEARCH REPORT**
Information on patent family members

International application No.

**PCT/CN2023/143105**

| Patent document cited in search report | | | Publication date (day/month/year) | Patent family member(s) | | | Publication date (day/month/year) |
|---|---|---|---|---|---|---|---|
| | | | | CA | 3049212 | A1 | 12 July 2018 |
| CN | 111566126 | A | 21 August 2020 | AR | 114080 | A1 | 22 July 2020 |
| | | | | KR | 20200110362 | A | 23 September 2020 |
| | | | | IL | 275794 | A | 31 August 2020 |
| | | | | RU | 2020126721 | A3 | 14 February 2022 |
| | | | | SG | 11202006610 | YA | 28 August 2020 |
| | | | | UY | 38050 | A | 31 July 2019 |
| | | | | MX | 2020006976 | A | 05 October 2020 |
| | | | | EP | 3737699 | A1 | 18 November 2020 |
| | | | | US | 2019218279 | A1 | 18 July 2019 |
| | | | | US | 10738110 | B2 | 11 August 2020 |
| | | | | US | 2022195022 | A1 | 23 June 2022 |
| | | | | US | 11891435 | B2 | 06 February 2024 |
| | | | | BR | 112020013621 | A2 | 01 December 2020 |
| | | | | CL | 2020001846 | A1 | 05 February 2021 |
| | | | | TW | 201940511 | A | 16 October 2019 |
| | | | | WO | 2019140216 | A1 | 18 July 2019 |
| | | | | CA | 3087951 | A1 | 18 July 2019 |
| | | | | AU | 2019207915 | A1 | 16 July 2020 |
| | | | | JP | 2023116808 | A | 22 August 2023 |
| | | | | US | 2021017261 | A1 | 21 January 2021 |
| | | | | US | 11248043 | B2 | 15 February 2022 |
| | | | | JP | 2021511024 | A | 06 May 2021 |
| | | | | JP | 7339262 | B2 | 05 September 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7229619 B

- HR 1214

**Non-patent literature cited in the description**

- Computational Molecular Biology. Oxford University Pres, 1988
- Biocomputing: Informatics and Genome Projects. Academic Press, 1993
- Computer Analysis of Sequence Data, Part I. Humana Press, 1994
- Sequence Analysis in Molecular Biology. Academic Press, 1987
- Sequence Analysis Primer. M Stockton Press, 1991
- **CARRILLO, H.** ; **LIPMAN, D.** *SIAM J Applied Math*, 1988, vol. 48, 1073

- **RICH** ; **MYSZKA**. *Curr. Opin. Biotechnol*, 2000, vol. 11, 54
- **ENGLEBIENNE**. *Analyst.*, 1998, vol. 123, 1599
- **BIACORE** ; **BIACORE AB** ; **UPSALA** ; **SWEDEN** ; **GE**. Healthcare Life Sciences; Malmqvist. *Biochem. Soc. Trans.*, vol. 27, 335
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc.Natl.Acad.Sci.USA*, 1997, vol. 94, 412-417